# EUROPEAN PATENT SPECIFICATION

(11) **EP 3 140 656 B1**
(45) Date of publication and mention of the grant of the patent: **17.02.2021**
(21) Application number: 15788742.3
(22) Date of filing: 08.05.2015
(51) Int. Cl.: G01N 33/574, G01N 33/50, G01N 33/569

(54) **COMPOSITIONS AND METHODS FOR FLUID BIOPSY OF MELANOMA**
ZUSAMMENSETZUNGEN UND VERFAHREN ZUR FLUIDBIOPSIE VON MELANOMEN
COMPOSITIONS ET PROCÉDÉS DE BIOPSIE DE LIQUIDES PERMETTANT LE DIAGNOSTIC DE MÉLANOME

(30) Priority: 09.05.2014 US 201461991088 P
(43) Date of publication of application: 15.03.2017
(73) Proprietor: The Scripps Research Institute, La Jolla, CA 92037 (US)
(72) Inventor: KUHN, Peter, Solana Beach, CA 92075 (US); VELASCO, Carmen, Ruiz, La Jolla, CA 92037 (US)
(74) Representative: Jones Day
(86) International application number: PCT/US2015/029914
(87) International publication number: WO 2015/172038

(56) References cited:
- WO-A1-2011/050103
- WO-A1-2013/111054
- US-A1- 2008 076 727
- US-A1- 2008 076 727
- US-A1- 2009 317 836
- US-A1- 2011 300 551
- TERSTAPPEN: "Circulating melanoma cells and survival in metastatic melanoma", INTERNATIONAL JOURNAL OF ONCOLOGY, vol. 38, no. 3, 1 March 2011 (2011-03-01), XP055408457, GR ISSN: 1019-6439, DOI: 10.3892/ijo.2011.896
- LEILA KHOJA ET AL: "Biomarker Utility of Circulating Tumor Cells in Metastatic Cutaneous Melanoma", THE JOURNAL OF INVESTIGATIVE DERMATOLOGY : OFFICIAL JOURNAL OF THE SOCIETY FOR INVESTIGATIVE DERMATOLOGY AND THE EUROPEAN SOCIETY FOR DERMATOLOGICAL RESEARCH, vol. 133, no. 6, 1 June 2013 (2013-06-01), pages 1582-1590, XP055408500, US ISSN: 0022-202X, DOI: 10.1038/jid.2012.468
- CAMPOLI M R ET AL: "HUMAN HIGH MOLECULAR WEIGHT-MELANOMA-ASSOCIATED ANTIGEN (HMW-MAA): A MELANOMA CELL SURFACE CHONDROITIN SULFATE PROTEOGLYCAN (MSCP) WITH BIOLOGICAL AND CLINICAL SIGNIFICANCE", CRITICAL REVIEWS IN IMMUNOL, CRC PRESS, INC, US, vol. 24, no. 4, 1 January 2004 (2004-01-01), pages 267-296, XP008066466, ISSN: 1040-8401, DOI: 10.1615/CRITREVIMMUNOL.V24.I4.40
- MARRINUCCI ET AL.: 'Fluid biopsy in patients with metastatic prostate, pancreatic and breast cancers' PHYS BIOL. vol. 9, no. 1, 03 February 2012, pages 1 - 19, XP020218196

## Description

The present invention relates generally to the field of cancer diagnostics, and more specifically to methods for diagnosing metastatic melanoma.

### BACKGROUND OF THE INVENTION

Circulating tumor cells (CTCs) released from either a primary tumor or its metastatic sites hold important information about the biology of the tumor. CTCs can be considered not only as surrogate biomarkers for metastatic disease but also as a promising key tool to track tumor changes, treatment response, cancer recurrence or patient outcome non-invasively. However, the extremely low levels of CTCs in the bloodstream combined with their unknown phenotype has significantly impeded their detection. Thus, the research to evaluate the clinical utility of CTCs has been limited. A variety of technologies have been developed over the past 20 years for specific isolation of CTCs in order to utilize their information.

In the context of melanoma, one of the more widely used techniques for detecting circulating melanoma cells (CMCs) is an RT-PCR method that detects the presence of tumor RNA in the bloodstream of melanoma patients. Recently, methodologies have been developed for detecting CMCs that rely on enrichment of CMCs in a sample by capturing the intact CMCs, allowing downstream molecular, morphologic and/or phenotypic characterization. One frequently used method depends on immunomagnetic enrichment of tumor cells from peripheral blood. However, one of the main limitations of this positive selection is that only CMCs with sufficient expression of the selected surface marker are detected. One negative selection approach relies on anti-CD45-coated immunomagnetic beads to selectively remove peripheral blood mononuclear cells (PBMCs). Still other enrichment-based methodologies rely on physical differences between PBMCs and CMCs such as size or density. However, technical inadequacies of these methodologies, which include low recovery rates, in combination with few suitable biomarkers that are expressed by all CMCs, has limited their adoption.

Thus, there exists a need for improved methods for CMC detection and characterization. The present disclosure addresses this need as well as providing related advantages.

Rao et al., 2011; Int. J Oncol, 38(3), 755-760 relates to an assay for circulating melanoma cells in which CD146+ cells are immunomagnetically enriched.

Khoja et al., 2013; J Invest Dermatol, 133(6), 1582-1590 relates to a study of the utility of circulating tumor cells as prognostic and pharmacodynamics biomarkers.

WO 2011/050103 relates to detection and categorization of rare cell, such as circulating tumor cells, in diagnostic methods.

Campoli et al., 2004; Crit Rev Immunol, 24(4), 267-296 is a review summarizing the characteristics of the HMW-MAA/MCSP in terms of structure, antigenic profile, tissue distribution, and similarities with its counterparts in other animal species.

US 2008/076727 relates to HMW-MAA antibody cocktails and their uses in detecting cancer and isolating cancer cells.

### SUMMARY OF INVENTION

The present invention provides methods for identifying CMCs in a biological sample and methods for diagnosing metastatic melanoma in a subject.

In a first aspect, the present invention provides a method for identifying circulating melanoma cells (CMCs) in a biological sample comprising: (a) contacting a biological sample of non-enriched blood with one or more detectable agents, wherein at least one of said one or more detectable agents is specific for a biomarker of CMCs, wherein the non-enriched sample is a sample not enriched for any specific population or subpopulation of nucleated cells; (b) determining the presence or absence of said one or more detectable agents in or on nucleated cells in the sample; and (c) assessing the morphology of the nucleated cells having said one or more detectable agents, wherein the CMCs are identified based on a combination of the presence or absence of said one or more detectable agents and morphological characteristics of the nucleated cells.

In a second aspect, the present invention provides a method for diagnosing metastatic melanoma comprising: (a) contacting a biological sample of non-enriched blood with one or more detectable agents, wherein said sample was obtained from a subject suspected of having metastatic melanoma or diagnosed with having melanoma, wherein at least one of said one or more detectable agents is specific for a biomarker of circulating melanoma cells (CMCs), wherein the non-enriched sample is a sample not enriched for any specific population or subpopulation of nucleated cells; (b) determining the presence or absence of said one or more detectable agents in or on nucleated cells present in the sample; (c) assessing the morphology of the nucleated cells having said one or more detectable agents; and (d) identifying the presence of CMCs in the sample based on a combination of the presence or absence of said one or more detectable agents and morphological characteristics of the nucleated cells, wherein the subject is diagnosed with metastatic melanoma when a predetermined number of CMCs is present in the sample.

In one embodiment, said one or more detectable agents comprise a immunofluorescent marker. In a further embodiment, said immunofluorescent maker is an antibody or functional fragment thereof that specifically binds to chondroitin sulfate proteoglycan 4 (CSPG4) or premelanosome protein (Pmel17), or S100 calcium-binding protein A1 (S100A1). In a further embodiment, said antibody is monoclonal. In another embodiment, said one or more detectable agents comprise two, three, four, five, six, seven or more immunofluorescent markers. In another embodiment, said one or more detectable agents comprise a nucleic acid specific stain. In a further embodiment, said nucleic acid specific stain is a Hoechst stain. In another embodiment, said one or more detectable agents comprise an immunofluorescent marker for white blood cells (WBCs). In a further embodiment, said immunofluorescent marker for WBCs is an antibody specific for cluster of differentiation 45 (CD45).

In another embodiment, step (b) and/or (c) are performed by automated fluorescent microscopy.

In another embodiment, said determining the presence or absence of said one or more detectable agents comprises comparing distinct immunofluorescent staining of CMCs with distinct immunofluorescent staining of white blood cells (WBCs). In a further embodiment, said immunofluorescent staining of CMCs is positive for an antibody or functional fragment thereof that specifically binds to CSPG4 and is detectable at a standard deviation of the mean (SDOM) of greater than or equal to 2. In a further embodiment, said immunofluorescent staining of CMCs is negative for an antibody or functional fragment thereof that specifically binds to CD45. In yet a further embodiment, said immunofluorescent staining of CMCs is positive for Hoeschst staining.

In another embodiment, said morphological assessment comprises comparing the morphological characteristics of CMCs with the morphological characteristics of surrounding white blood cells (WBCs). In a further embodiment, said morphological characteristics comprise nucleus size, nucleus shape, cell size, cell shape or nuclear to cytoplasmic ratio. In yet a further embodiment, a nuclear to cytoplasmic ratio of less than 2.5 indicates the presence of a CMC.

In another embodiment, the methods of the first and second aspect may further comprise obtaining a white blood cell (WBC) count for the sample. In another embodiment, the methods of the first and second aspect may further comprise lysing erythrocytes in the sample. In another embodiment, the methods of the first and second aspect may further comprise depositing nucleated cells from the sample as a monolayer on a glass slide. In a further embodiment, the methods may comprise depositing about 3 million cells from the sample onto said glass slide. In another embodiment, said predetermined number of CMCs is at least 0.5, 1.0, 1.5, 2.0, 2.5, 3.0, 4.0, 5.0, 10, 20, 50, 100, 200, 300, 400 or 500 CMCs per ml of sample.

Other features and advantages of the invention will be apparent from the detailed description, and from the claims.

### BRIEF DESCRIPTION OF THE DRAWINGS

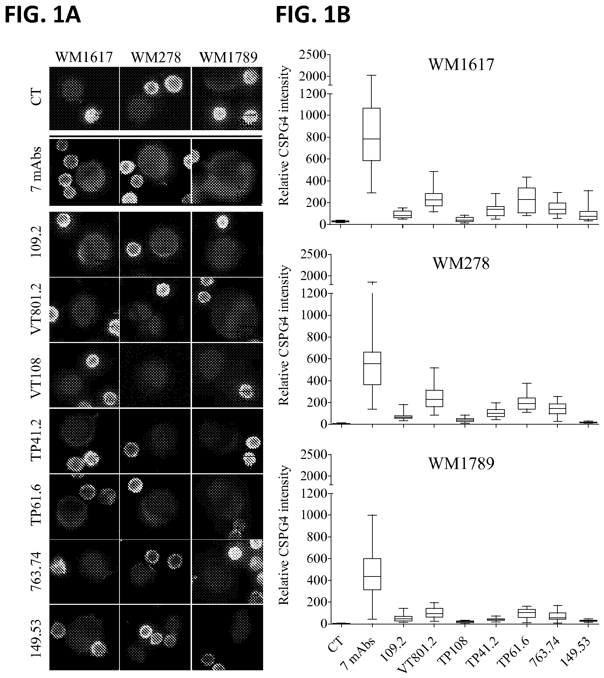

**FIGs. 1A-1B** show CSPG4 expression in melanoma cells. (**FIG. 1A**) Representative merged images of WM1617, WM278 and WM1789 melanoma cells stained with CSPG4-specific mAbs are shown in column 1, 2 and 3 (from left to right), respectively. Controls are shown in line 1, the combination of the 7 CSPG4-specific mAbs is shown in line 2 and the use of each CSPG4-specific mAb individually is shown from line 3 to 9. Light grey is CSPG4. (**FIG. 1B**) Relative CSPG4 intensity normalized against the background signal on PBMCs in WM1678, WM278 and WM1789 melanoma cell lines.

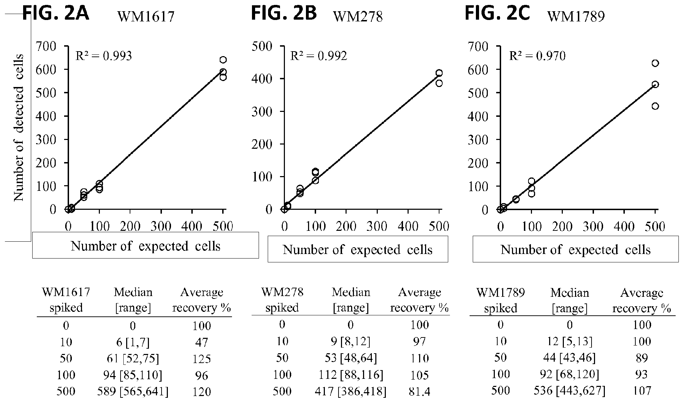

**FIGs. 2A-2C** show the detection of melanoma cells spiked into PBMCs from normal blood. Melanoma cells from 3 melanoma cells lines (**FIG. 2A** - WM1617, **FIG. 2B** **-** WM278 and **FIG. 2C** - WM1789) were spiked at different concentrations into PBMCs from a normal blood donor to produce 6 slides in triplicate with 0, 10, 25, 50, 100 and 500 melanoma cells per slide and for each melanoma cell line. The expected number of cells spiked is plotted versus the number of cells detected.

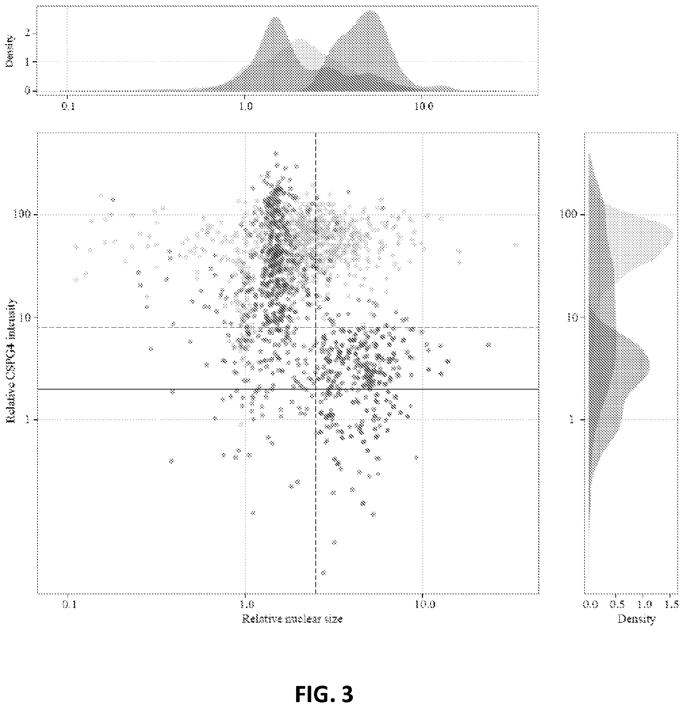

**FIG. 3** shows a scatter plot of candidate cells used to define CMCs. Ten normal blood donors, 40 melanoma patients and 3 melanoma cell lines classified by relative CSPG4 protein expression and relative nuclear size are shown. Both measurements are normalized against the values obtained in surrounding PBMCs. Cells from melanoma patients, normal donors and cell lines are represented in black, light grey and grey, respectively.

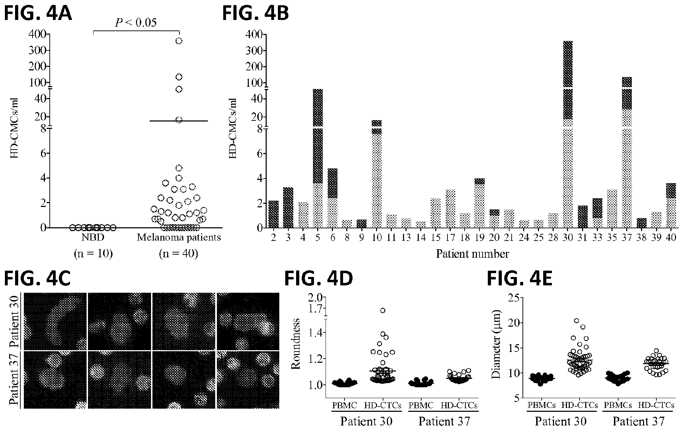

**FIGs. 4A-4E** show the prevalence of CMCs in metastatic melanoma patients. (**FIG. 4A**) Metastatic melanoma patients (n = 40) and normal blood donors (n = 10) were evaluated using the HD-CTC platform in combination with a panel of 7 mAbs. The mean values are shown as a plain black line. P < 0.05 Wilcoxon t test. (**FIG. 4B**) HD-CMC/ml was calculated for each patient. Dark bars indicate the amount of CSPG4 bright HD-CMCs and light bars indicate the amount of dim CSPG4 HD-CMCs detected per melanoma patient. (**FIG. 4C**) Representative merged images of 8 HD-CMCs from 2 melanoma patients. Hoechst staining is represented in dark grey, CSPG4 in light grey and CD45 in bright grey. (**FIG. 4D**) Roundness (perimeter squared)/(4^{∗}pi^{∗}area), with 1 indicating perfect circle and larger values indicating oblong objects. (**FIG. 4E**) Area comparisons between PBMCs and HD-CMC from melanoma patients. The mean values are shown as a line. Error bars represent the standard deviation.

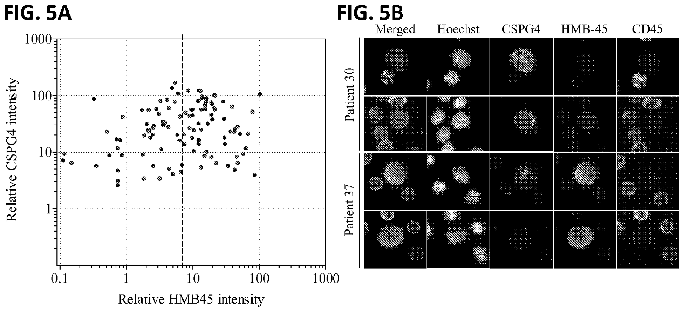

**FIGs. 5A-5B** show the characterization of CMCs with HMB45. (**FIG. 5A**) Scatter plot of HD-CMCs detected in 40 melanoma patients classified by relative CSPG4 signal intensity and relative HMB-45 signal intensity. Both measurements are normalized against the values obtained in surrounding PBMCs. Vertical dashed line indicate the cutoff value for HMB-45 positive signal. (**FIG. 5B**) Gallery of 4 HD-CMC detected in two melanoma patients (patient 30 and 37) using the HD-CMC assay in combination with HMB-45 staining.

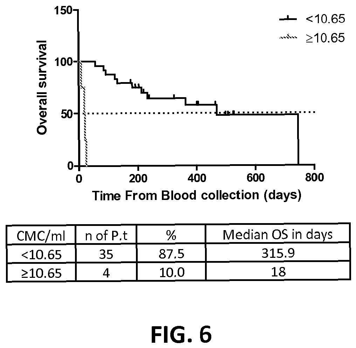

**FIG. 6** shows the relationship between CMC levels and the overall survival of melanoma patients.

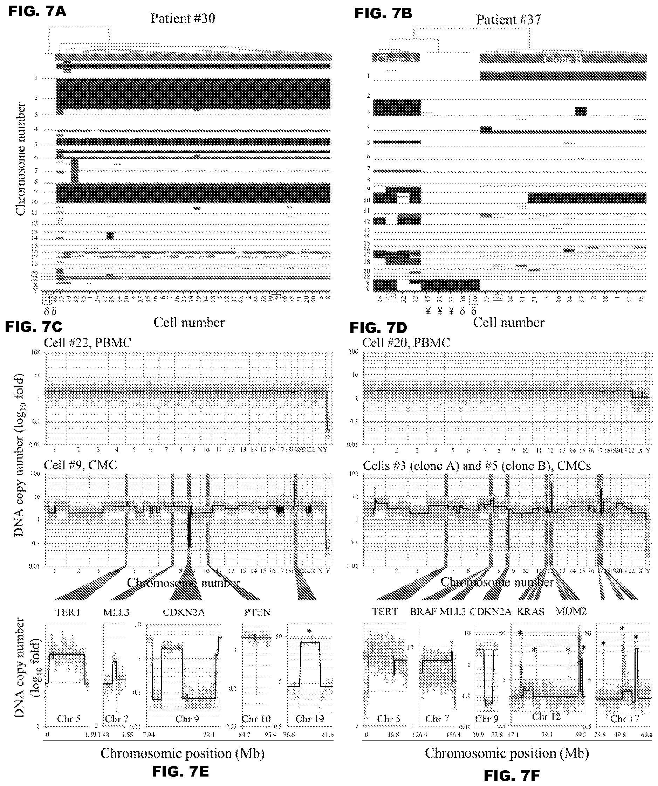

**FIGs. 7A-7F** show DNA copy number variations in single CMCs isolated from two melanoma patients. (**FIG. 7A and 7B**) Heatmaps representing chromosomic gains (light grey) and deletions (black) in single CMCs from patient #30 and patient #37; the hierarchical clustering was performed in R using the heatmap.2 function in the gplots package. Ward's method with Manhattan distance metric was used for the clustering. Using median centered CNV profiles, cutoff ratios versus the median of 0.675 and 1.7 were used to define deletions and amplifications, respectively. These cutoffs were used both to color the heatmap and to do the frequency analysis (FIG. 7C and 7D). (**FIG. 7C** and **7D**) Representative single PBMCs (top) and CMCs (bottom) DNA CNV profiles. Solid and dashed lines in (FIG. 7D) (bottom) and (FIG. 7F) represents clone A and B, respectively. Adjusted log10 ratio of read depth of sequencing data are plotted for individual bins (y axis) across genomic regions (x axis). (**FIG. 7E** and **7F**) Candidate genes located in the amplified and deleted genomic regions. PMBCs (δ), 'excluded candidate' cells (¥) and cells displayed in detail in (FIG. 7C) and (FIG. 7D) ( ). Novel chromosomal amplifications (*).

### DETAILED DESCRIPTION OF THE INVENTION

The present disclosure is based, in part, on the unexpected discovery that CMCs can be detected and identified in a biological sample, such as a non-enriched blood sample, by determining the presence or absence of a detectable agent specific for a CMC biomarker and assessing the morphology of the CMCs in the sample. The present disclosure is further based, in part, on the discovery that the presence of CMCs in a sample can be a diagnostic indicator of metastatic melanoma. As described herein, CMCs were identified in metastatic melanoma patients. Additionally, despite CMCs having a heterogeneous population in terms of biomarker expression and cell morphology within and across melanoma patients, use of a combination of immunofluorescent markers, which increased the intensity of the staining of melanoma cell lines, and certain morphological characteristics of CMCs, provided for a method of identifying CMS in a biological sample.

A fundamental aspect of the present disclosure is the robustness of the disclosed methods. The rare event detection (RED) disclosed herein with regard to CMCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis but that instead compare enriched populations with inherently distorted contextual comparisons of rare events.

Provided herein are methods for identifying CMCs in a biological sample and for diagnosing subjects with metastatic melanoma. One major advantage of the present methods disclosed is the surprisingly high sensitivity with which the methods can classify subjects into a subject suffering from metastatic melanoma or a healthy subject, especially in non-enriched blood samples having very low CMC counts. High classification sensitivities at low CMC counts facilitates the detection and diagnosis, and thereby facilitating the timely treatment of a subject. The present disclosure is therefore of particular benefit to a subject who is at an elevated risk of developing melanoma, *e.g.*, due to a genetic predisposition for melanoma, sun exposure, exposure to environmental factors, pigmentary characteristics, immunosupression, family history of melanoma or personal history of melanoma or non-melanoma skin cancer.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The term "biological sample" refers to any specimen from the body of an organism that can be used for analysis or diagnosis. In the context of the present disclosure, a biological sample can be a sample that contains or is suspected to contain CMCs. A biological sample obtained from a subject can be any sample that contains or is suspected to contain cells and encompasses any material in which CMCs can be detected. For example, a biological sample can include a solid tissue sample (*e.g.*, bone marrow) or a liquid sample (*e.g.,* blood, whole blood, plasma, amniotic fluid, pleural fluid, peritoneal fluid. central spinal fluid, urine, saliva or other body fluid that contains cells). As provided by the invention, the biological sample is a non-enriched blood sample. As described herein, a preferred sample is a whole blood sample, more preferably a peripheral blood sample. As will be appreciated by those skilled in the art, a blood sample can include any fraction or component of blood including, without limitation, T-cells, monocytes, neutrophiles, erythrocytes, platelets and microvesicles, such as exosomes and exosome-like vesicles. In the context of this disclosure, blood cells included in a blood sample can encompass any nucleated cells and are not limited to components of whole blood. As such, blood cells include, for example, both white blood cells (WBCs) as well as rare cells, including CMCs.

The phrase "non-enriched blood sample" refers to a blood sample that has not undergone a process so as to substantially add or increase the proportion of target cells or molecules in the sample. The target cells can be, for example. CMCs in the context of the present disclosure. Accordingly, in the invention a non-enriched sample is a sample that is not enriched for any specific population or subpopulation of nucleated cells. For example, a non-enriched blood sample may not be enriched for CMCs, WBC, B-cells, T-cells, NK-cells, monocytes, or the like. The proportion of target cells or molecules can also be relative to another component in the sample (*e.g.*, WBCs, red blood cells (RBCs), platelets, plasma, or any other molecule known to be present in blood). Several processes that can add or increase the proportion of target cells in a sample, including both positive and negative selection methods, are known in the art. For example, a non-enriched blood sample can include a sample that has not undergone: a positive selection for CMCs, such as, for example, enrichment by antibody binding to CMCs or epithelial cells (*e.g.,* EpCAM MicroBeads) or immunomagnetic enrichment (*e.g.*, magnetic-activated cell sorting (MACs), ferrofluids of coated antibodies that bind CMCs, or antibody coated magnetic beads that bind to CMCs); a negative selection, such as, for example, size filtration (*e.g*., passage through an 8 µm filter), depletion of hematopoietic cells by antibody binding (*e.g.*, anti-CD45 coated magnetic beads) or density gradient centrifugation; or any combination of both positive and negative selection. Accordingly, a non-enriched blood sample useful in the described methods can be a sample that has not undergone a positive and/or negative selection process. As will be appreciated by those skilled in the art, a non-enriched blood sample can include a blood sample that has undergone a process that does not substantially add or increase the proportion of target cells or molecules in the sample. Such processes can include, for example, addition of a preservative (*e.g*., anticoagulant, buffer, adenine, sodium phosphate, citric acid, dextrose, mannitol, sodium chloride), addition of a cyroprotectant (*e.g.*, glycerol), or lysis of red blood cells (*e.g.*, addition of ammonium chloride).

The samples of this disclosure can each contain a plurality of cell populations and cell subpopulation that are distinguishable by methods well known in the art (*e.g.,* FACS, immunohistochemistry). For example, a blood sample can contain populations of non-nucleated cells, such as erythrocytes (*e.g*., 4-5 million/µl) or platelets (150,000-400,000 cells/µl), and populations of nucleated cells such as WBCs (*e.g.,* 4,500 - 10,000 cells/µl) and CMCs *(e.g.,* 1-800 cells/ml). WBCs may contain cellular subpopulations of, *e.g.,* neutrophils (2,500-8,000 cells/µl), lymphocytes (1,000-4,000 cells/µl), monocytes (100-700 cells/µl), eosinophils (50-500 cells/µl), basophils (25 - 100 cells/ µl) and the like.

The biological samples of this disclosure may be obtained from any organism, including mammals such as humans, primates (*e.g.*, monkeys, chimpanzees, orangutans, and gorillas), cats, dogs, rabbits, farm animals *(e.g.*, cows, horses, goats, sheep, pigs), and rodents *(e.g.,* mice, rats, hamsters, and guinea pigs).

It is noted that, as used herein, the terms "subject," "organism," "individual" or "patient" are used as synonyms and interchangeably, and refers to a vertebrate, preferably a mammal. Mammals include, but are not limited to, humans, primates (*e.g.*, monkeys, chimpanzees, orangutans, and gorillas), cats, dogs, rabbits, farm animals (*e.g.*, cows, horses, goats, sheep, pigs), and rodents (*e.g.*, mice, rats, hamsters, and guinea pigs).

The subjects of this disclosure include, for example, any subject having: melanoma (*e.g.*, diagnosed with melanoma); suspected of having melanoma; suspected of having metastatic melanoma; or being at risk of developing melanoma. Elevated risks for developing melanoma can be due to a genetic predisposition for melanoma (*e.g.*, cyclin-dependent kinase inhibitor 2A (CDKN2A) mutations, mutations in the promoter region of a subunit of telomerase reverse transcriptase (TERT), cyclin-dependent kinase 4 (CDK4) mutations, cyclin-dependent kinase 6 (CDK6) mutations, xeroderma pigmentosum (XP) patients, Cowden syndrome/PTEN hamartoma tumor syndrome (PHTS) patients; mutations in melanoma susceptibility locus on 1p22, alpha melanocyte-stimulating hormone receptor (MC1R) mutations, microphthalmia-associated transcription factor (MITF) mutations, BRCA2 mutations), certain sun exposure (*e.g.*, chronic sun exposure), exposure to environmental factors (*e.g.*, solvents, ionizing radiation, electromagnetic fields, vinyl chloride, polychlorinated biphenyls (PCBs)), certain pigmentary characteristics (*e.g.*, low scores on the Fitzpatrick skin types I-VI), nevi (*i.e.,* birthmarks or beauty marks), immunosupression, family history of melanoma or personal history of melanoma or non-melanoma skin cancer. In some aspects, the subject is or has been a cancer patient (*e.g.*, melanoma, skin cancer), received an anti-cancer treatment, or discontinued an anti-cancer treatment. Anti-cancer treatments include, for example and without limitation, surgery, drug therapy (*e.g.*, chemotherapy), radiation therapy, or combinations thereof. In some aspects, the subject is treatment naive.

The subject can be a healthy organism, including, for example and without limitation, an individual or a non-cancer patient in the control group of a clinical study, a cured cancer patient or an individual at risk of developing cancer.

The subject can also be an animal model for cancer, including, without limitation, a xenograft mouse model, a transgenic mouse carrying a transgenic oncogene, a knockout mouse lacking a proapoptotic gene and others. A person of skilled in the art understands that many other animal models for cancer conditions (*e.g.*, mice or other organisms) are well known in the art and can be the subject of the methods disclosed herein.

"Melanoma" refers to a form of skin cancer that originates in the pigment-producing cells (melanocytes) of the basal layer of the epidermis. Melanoma can also involve the colored part of the eye or the bowel. As one skilled in the art would understand, melanoma can originate in any part of the body that contains melanocytes.

"Metastatic melanoma," also known as stage IV melanoma, refers to when melanoma cells of any kind have spread through the lymph nodes to distant sites in the body and/or to the body's organs. Organs that are frequently affected by metastatic melanoma include the liver, lungs, bones and brain (Fiddler, Cancer Control. 1995 Oct;2(5):398-404).

The "circulating tumor cells" or "CTCs" of this disclosure are tumor cells that are circulating in the bloodstream of an organism.

The "circulating melanoma cells" or "CMCs" of this disclosure are melanoma cells that are circulating in the bloodstream of an organism.

The term "detectable agent" or "detectable label" refers to a molecule that can be used for the direct or indirect detection of a biomarker. A wide variety of detectable agents are known in the art and can be readily identified and used by a person skilled in the art. Suitable detectable agents include, but are not limited to, fluorescent dyes (*e.g.*, fluorescein, fluorescein isothiocyanate (FITC), Oregon Green™, rhodamine, Texas Red, tetrarhodamine isothiocynate (TRITC), Cy3, Cy5, Alexa Fluor® 647, Alexa Fluor® 555, Alexa Fluor® 488), fluorescent protein markers (*e.g.*, green fluorescent protein (GFP), phycoerythrin, etc.), enzymes (*e.g.*, luciferase, horseradish peroxidase, alkaline phosphatase, etc.), nanoparticles, biotin, digoxigenin, metals, and the like.

The term "immunofluorescent marker" refers to a detectable agent that is an antibody or functional fragment thereof that targets a fluorescent dye to a specific molecule within or on a cell. An immunofluorescent marker can be used in methods that employ a fluorescent light microscope to produce immunostaining for a desired sample. An immunofluorescent marker can also be employed in immunocytochemistry (ICC) or immunohistochemistry (IHC) methods described herein. In the context of the present disclosure, an immunofluorescent marker can be used to detect a CMC as described herein.

The term "antibody" refers to any immunoglobulin or derivative thereof, whether natural or wholly or partially synthetically produced. All antibody derivatives which maintain specific binding ability can also be used in the disclosed methods. The antibodies of this disclosure can bind specifically to a biomarker. For example, the antibodies can bind specifically to a single biomarker (*e.g.*, chondroitin sulfate proteoglycan 4 (CSPG4)). Additionally, the antibodies can be pan-specific. For sample, pan-specific antibodies of this disclosure can bind specifically to one or more members of a biomarker family (*e.g.,* one or more members of the chondroitin sulfate proteoglycan family, including chondroitin sulfate proteoglycan 1, 2, 3, 4, 5, 6, 7 and 8). The antibody can have a binding domain that is homologous or largely homologous to an immunoglobulin binding domain and can be derived from natural sources, or partly or wholly synthetically produced. The antibody can be a monoclonal or polyclonal antibody. In some aspects, the antibody is a single-chain antibody. In some aspects, the antibody includes a single-chain antibody fragment. In some aspects, the antibody can be an antibody fragment including, but not limited to, Fab, Fab', F(ab')2, scFv, Fv, dsFv diabody, and Fd fragments. Due to their smaller size antibody fragments can offer advantages over intact antibodies in certain applications. Alternatively or additionally, the antibody can comprise multiple chains which are linked together, for example, by disulfide linkages, and any functional fragments obtained from such molecules, wherein such fragments retain specific-binding properties of the parent antibody molecule. Those of skill in the art will appreciate that the antibody can be provided in any of a variety of forms including, for example, humanized, partially humanized, chimeric, chimeric humanized, *etc.* The antibody can be prepared using any suitable methods known in the art. For example, the antibody can be enzymatically or chemically produced by fragmentation of an intact antibody or it can be recombinantly produced from a gene encoding the partial antibody sequence.

The term "biomarker" refers to a biological molecule, or a fragment of a biological molecule, the change and/or the detection of which can be correlated with a particular physical condition or state of a CMC. The terms "marker" and "biomarker" are used interchangeably throughout the disclosure. Such biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.*, glycoproteins, ribonucleoproteins, lipoproteins). The term also encompasses portions or fragments of a biological molecule, for example, peptide fragment of a protein or polypeptide. In the context of the present disclosure, exemplary biomarkers for CMCs include chondroitin sulfate proteoglycan 4 (CSPG4), premelanosome protein (Pmel17) and S100 calcium-binding protein A1 (S100A1).

"Chondroitin sulfate proteoglycan 4" or "CSPG4," also known as high molecular weight melanoma associated antigen (HMW-MAA) and melanoma chondroitin sulfate proteoglycan (MCSP), is a membrane-bound proteoglycan that mediates both cell-cell and cell-extracellular matrix interactions and has been associated with the metastatic potential of melanoma cells (Price et al., Pigment Cell Melanoma Res. 2011 Dec; 24(6):1148-57; Yang et al., J Cell Biol. 2004 Jun 21;165(6):881-91; Yang et al., Cancer Res. 2009 Oct 1;69(19):7538-47; Iida et al., J Biol Chem. 2001 Jun 1;276(22):18786-94).

"Premelanosome protein" or "Pmel17," also know as Silver, SILV, GP100 and ME20, refers to is a 100 kDa type I transmembrane glycoprotein that is expressed primarily in pigment cells of the skin and eye and is responsible for the formation of fibrillar sheets within the pigment organelle, the melanosome (Kim et al., Pigment Cell Res. 1996 Feb;9(1):42-8; Watt et al., Pigment Cell Melanoma Res. 2013 May;26(3):300-15). HMB-45 is a monoclonal antibody that specifically reacts against Pmel 17, and stands for Human Melanoma Black (Gown et al., Am J Pathol. 1986 May;123(2):195-203). HMB-45 can be used in anatomic pathology as a marker for melanoma (Mahnood et al., Mod Pathol. 2002 Dec;15(12):1288-93).

"S100 calcium-binding protein A1" or "S100A1" refers to a member of the S100 family of proteins containing four EF-hand calcium-binding motifs in its dimerized form, which in humans is encoded by the S100A1 gene (Marenholz et al., Biochem Biophys Res Commun. 2004 Oct 1;322(4):1111-22; Morii et al., Biochem Biophys Res Commun. 1991 Feb 28;175(1):185-91). S100 proteins are localized in the cytoplasm and/or nucleus of a wide range of cells, and involved in the regulation of a number of cellular processes such as cell cycle progression and differentiation. S100A1 may function in stimulation of Ca2+-induced Ca2+ release, inhibition of microtubule assembly, and inhibition of protein kinase C-mediated phosphorylation. S100A1 expression has been seen in malignant melanomas in a diffuse reaction (Nonaka et al., J Cutan Pathol. 2008 Nov;35(11):1014-9).

"Morphology" or "morphological characteristic," when used in reference CMCs, refers to a feature, form or structure of CMCs that is shared between CMCs. Examples of such features, forms or structures include, without limitation, the presence of an intact nucleus, the nucleus size, the nucleus shape, the cell size, the cell shape and the nuclear to cytoplasmic ratio. Accordingly, in some aspects, for example, the morphology indicative of a CMC is a nuclear to cytoplasmic ratio of less than 4.0, 3.5, 3.0, 2.5, 2.0, 1.5, or 1.0.

As used herein, the term "cluster" means two or more CMCs with touching cell membranes.

"Nucleic acid specific stain" refers to a molecule that selectively binds to a nucleic acid in a sample and produces a distinctive detectable signal, either directly or indirectly. A nucleic acid specific stain includes, but is not limited to, a molecule that binds to double stranded deoxyribonucleic acids (DNA) via intercalation, major groove binding, minor groove binding, external binding or bis-intercalation. Examples of intercalating molecules include ethidium bromide and propidium iodide. Examples of minor-groove binders include 4',6-diamidino-2-phenylindole (DAPI) and bis-benzimides dyes (also known as Hoechst dyes) (*e.g.,* Hoechst 33258, Hoechst 33342, and Hoechst 34580). Examples of other nucleic acid stains include acridine orange, 7-aminoactinomycin D (7-AAD), SYTOX Blue, Chromomycin A3, Mithramycin, YOYO-1, SYTOX Green, TOTO-1, TO-PRO-1, TO-PRO: Cyanine Monomer, Thiazole Orange, CyTRAK Orange, LDS 751, SYTOX Orange, TOTO-3, TO-PRO-3, DRAQ5, and DRAQ7.

"Automated fluorescent microscopy" refers to a system of operating and/or controlling an optical microscope that uses fluorescence or phosphorescence by automatic devises to generate an image of a sample thereby reducing human intervention to a minimum. Such a system can include capturing images of the sample and analyzing the images to identify the presence or absence of CMC's in the sample.

As used herein, the term "predetermined number" when used in reference to the amount of CMCs relative to the amount of another compound or sample volume is intended to mean a number of CMCs that is established in advance of performing a method described herein that is indicative of a subject having metastatic melanoma. The predetermined number can be identified through prior experimental observations. In the context of the present disclosure, a predetermined number of CMCs present in a sample that is at least 0.5, 1.0, 1.5, 2.0, 2.5, 3.0, 4.0, 5.0, 10, 20, 50, 100, 200, 300, 400 or 500 CMCs per ml of sample can be indicative of a subject having metastatic melanoma. In another embodiment, a predetermined number of CMCs indicative of a subject having metastatic melanoma can be the number of CMCs per another component of the sample, such as, but not limited to, WBCs. The number of WBCs in the blood of a normal individual can be between about 4.0 x 10⁹ to about 12 x 10⁹ WBCs per liter of blood depending upon a number of factors including age, gender, and ethnicity, with the average individual after age 11 years having about 6.0-7.5 x 10⁹ WBCs per liter (Vital and Health Statistics, Series 11, No. 247 (March 2005). Accordingly, in some embodiments, the ratio of CMCs per WBCs in the sample can be indicative of a subject having metastatic melanoma. For example, the number of CMCs per WBCS can be 0.5, 1.0, 1.5, 2.0, 2.5, 3.0, 4.0, 5.0, 10, 20, 50, 100, 200, 300, 400 or 500 CMCs per 6.0-7.5 x 10⁶ WBCs.

Provided herein is a method for identifying CMCs in a biological sample. The method can include the steps of: (a) contacting a biological sample of non-enriched blood with one or more detectable agents, wherein at least one of the one or more detectable agents is specific for a biomarker of CMCs; (b) determining the presence or absence of the one or more detectable agents in or on nucleated cells in the sample; and (c) assessing the morphology of the nucleated cells having the one or more detectable agents, wherein the CMCs are identified based on a combination of the presence or absence of the one or more detectable agents and morphological characteristics of the nucleated cells.

Provided herein is a method for diagnosing metastatic melanoma. The method for diagnosing metastatic melanoma can include the steps of: (a) contacting a biological sample of non-enriched blood with one or more detectable agents, wherein the sample was obtained from a subject suspected of having metastatic melanoma or diagnosed with having melanoma, wherein at least one of the one or more detectable agents is specific for a biomarker of CMCs; (b) determining the presence or absence of the one or more detectable agents in or on nucleated cells present in the sample; (c) assessing the morphology of the nucleated cells having the one or more detectable agents; and (d) identifying the presence of CMCs in the sample based on a combination of the presence or absence of the one or more detectable agents and morphological characteristics of the nucleated cells, wherein the subject is diagnosed with metastatic melanoma when a predetermined number of CMCs is present in the sample.

One or more detectable agents are used in the methods of the invention. In some embodiments, two, three, four, five, six, seven, eight, nine, ten or more detectable agents can be used in the claimed methods. In some embodiments, two detectable agents can be used in the claimed methods. In some embodiments, three detectable agents can be used in the claimed methods. In some embodiments, four detectable agents can be used in the claimed methods. In some embodiments, five detectable agents can be used in the claimed methods. In some embodiments, six detectable agents can be used in the claimed methods. In some embodiments, seven detectable agents can be used in the claimed methods. In some embodiments, eight detectable agents can be used in the claimed methods. In some embodiments, nine detectable agents can be used in the claimed methods. In some embodiments, ten or more detectable agents can be used in the claimed methods. As will be understood by a person skilled in the art, when there are two or more agents used in a method of the disclosure the selection of the detectable agents can be dependent upon specific features and/or characteristics of the individual detectable agents. For example, in some embodiments, if two or more of the detectable agents are detectable by fluorescence, the specific excitation wavelength and/or emission wavelength of the fluorophores for each detectable agent will not overlap. Accordingly, a person skilled in the art would be able to readily ascertain which combination of detectable agents can be used in combination for the disclosed methods.

In some embodiments, the one or more detectable agents used in the methods can be an immunofluorescent marker, and in particular embodiments, the immunofluorescent marker specifically binds a biomarker of CMCs. An example of such an immunofluorescent marker includes, but is not limited to, an antibody or functional fragment thereof that specifically binds to CSPG4, Pmel17 or S100A1. In some embodiments, the antibody is monoclonal. In some embodiments, the one or more detectable agents can include two, three, four, five, six, seven or more immunofluorescent markers. For example, as disclosed herein, the use of multiple immunofluoresent markers, even directed to the same biomarker, can increase the sensitivity of the methods disclosed. Accordingly, in some embodiments, the one or more detectable agents include two immunofluorescent markers. In some embodiments, the one or more detectable agents include three immunofluorescent markers. In some embodiments, the one or more detectable agents include four immunofluorescent markers. In some embodiments, the one or more detectable agents include five immunofluorescent markers. In some embodiments, the one or more detectable agents include six immunofluorescent markers. In some embodiments, the one or more detectable agents include seven or more immunofluorescent markers. As will be understood by a person skilled in the art, when multiple immunofluorescent markers that target the same biomarker are used in the methods disclosed, it may be desirable for the markers to specifically recognize distinct and/or distant epitopes of the target biomarker. Accordingly, selection of immunofluorescent markers that are complementary to each other (*e.g.,* do not competitively inhibit binding to the target biomarker) can be selected by a person skilled in the art.

In some embodiments of the disclosure, the one or more detectable agents used in the disclosed method is a nucleic acid specific stain. For example, in some embodiments, the nucleic acid specific stain is a Hoechst stain or any other nucleic acid specific stain disclosed herein or well known in the art. As will be appreciated by a person skilled in the art, the selection of a nucleic acid specific stain can be dependent upon specific features and/or characteristics of the other detectable agents used in the disclosed method. A person skilled in the art could readily ascertain which nucleic acid specific stain would be compatible with the other detectable agents.

In some embodiments of the disclosure, the one or more detectable agents comprise an immunofluorescent marker specific for a component of a blood sample other than the CMCs. In some embodiments, the immunofluorescent marker is specific for WBCs. Such an WBC specific maker can be an antibody specific for cluster of differentiation 45 (CD45).

In some embodiments of the disclosure, the one or more detectable agents used in the disclosed methods can identify a CMC present in the sample even in the presence of other nucleated cells. In some embodiments, the immunofluorescent staining of CMCs is negative for an antibody or functional fragment thereof that specifically binds to CD45 (*i.e.,* CD45 (-)). However, the surrounding WBCs can be CD45 (+). In some embodiments, the immunofluorescent staining of CMCs is positive for Hoeschst staining (*i.e.,* Hoechst stain (+)). In some embodiments, the immunofluorescent staining of CMCs is positive for CSPG4 (*i.e.,* CSPG4 (+)). Accordingly, in particular embodiments, all nucleated cells are retained and immunofluorescently stained with one or more antibodies targeting a CMC biomarker (*e.g.*, CSPG4, Pmel17 or S100A1), an antibody targeting the common leukocyte antigen CD45, and a nucleic acid specific stain (e.g., Hoechst stain). The nucleated blood cells can be imaged in multiple fluorescent channels to produce high quality and high resolution digital images that retain fine cytologic details of nuclear contour and cytoplasmic distribution. While the surrounding WBCs can be identified with the antibody targeting CD45, the CMCs can be identified as, for example, CSPG4 (+), Hoechst stain (+) and CD45 (-). Accordingly, in the methods described herein, the CMCs can comprise distinct immunofluorescent staining from surrounding nucleated cells.

In some embodiments of the disclosure, the immunofluorescent staining of CMCs is positive for an antibody or functional fragment thereof that specifically binds to a CMC biomarker (e.g., CSPG4, Pmel17 or S100A1) based on a predetermine threshold intensity of fluorescence upon which to classify a candidate cell as being positive for CSPG4. For example, identifying a CMC as being positive for CSPG4 can be a candidate cell having a standard deviation of the mean (SDOM) of greater than or equal to 2. Accordingly, in some embodiments, the immunofluorescent staining of CMCs is positive for an antibody or functional fragment that specifically binds to CSPG4 or another CMC biomarker and is detectable at an SDOM of greater than or equal to 2, 2.5, 3, 3.5, 4, 4.5, 5, 6, 7, 8, 9 or 10. In some embodiments, the immunofluorescent staining of CMCs is detectable at an SDOM of greater than or equal to 2. In some embodiments, the immunofluorescent staining of CMCs is detectable at an SDOM of greater than or equal to 2.5. In some embodiments, the immunofluorescent staining of CMCs is detectable at an SDOM of greater than or equal to 3. In some embodiments, the immunofluorescent staining of CMCs is detectable at an SDOM of greater than or equal to 3.5. In some embodiments, the immunofluorescent staining of CMCs is detectable at an SDOM of greater than or equal to 4. In some embodiments, the immunofluorescent staining of CMCs is detectable at an SDOM of greater than or equal to 4.5. In some embodiments, the immunofluorescent staining of CMCs is detectable at an SDOM of greater than or equal to 5. In some embodiments, the immunofluorescent staining of CMCs is detectable at an SDOM of greater than or equal to 6. In some embodiments, the immunofluorescent staining of CMCs is detectable at an SDOM of greater than or equal to 7. In some embodiments, the immunofluorescent staining of CMCs is detectable at an SDOM of greater than or equal to 8. In some embodiments, the immunofluorescent staining of CMCs is detectable at an SDOM of greater than or equal to 9. In some embodiments, the immunofluorescent staining of CMCs is detectable at an SDOM of greater than or equal to 10.

In some embodiments, the presence or absence of a detectable agent, including an immunofluorescent marker, in or on nucleated cells, such as CMCs or WBCs, can result in distinct immunofluorescent staining patterns. Using the detectable agents disclosed herein, the methods of the disclosure can identify CMCs is a biological sample by determining the presence or absence of the one or more detectable agents on or in the candidate cells by comparing distinct immunofluorescent staining of CMCs with distinct immunofluorescent staining of WBCs. Immunofluorescent staining patterns for CMCs and WBCs may differ based on which markers are detected in the respective cells. In some embodiments, determining presence or absence of one or more immunofluorescent markers includes comparing the distinct immunofluorescent staining of CMCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, the method further includes analyzing the nucleated cells by nuclear detail, nuclear contour, presence or absence of nucleoli, quality of cytoplasm, quantity of cytoplasm, intensity of immunofluorescent staining patterns. A person skilled in the art understands that the morphological characteristics of this disclosure may include any feature, property, characteristic, or aspect of a cell that can be determined and correlated with the detection of a CMC.

In some embodiments of the disclosure, the methods include assessing the morphology of the nucleated cells having the one or more detectable agents as described herein *(e.g.,* cells that are CSPG4 (+), Hoechst stain (+) and CD45 (-)). Assessing the morphology of the cells in the biological sample can include, in some embodiments, comparing the morphological characteristics of CMCs with the morphological characteristics of surrounding WBCs. For example, the morphological characteristics that are compared can include nucleus size, nucleus shape, cell size, cell shape, and/or nuclear to cytoplasmic ratio. In some embodiments of the disclosure, a nuclear to cytoplasmic ratio of less than 5.0, 4.0, 3.5, 3.0, 2.5, 2.0, 1.5 or 1.0 can indicate the presence of a CMC. In some embodiments, the nuclear to cytoplasmic ratio indicating the presence of a CMC is less than 5.0. In some embodiments, the nuclear to cytoplasmic ratio indicating the presence of a CMC is less than 4.0. In some embodiments, the nuclear to cytoplasmic ratio indicating the presence of a CMC is less than 3.5. In some embodiments, the nuclear to cytoplasmic ratio indicating the presence of a CMC is less than 3.0. In some embodiments, the nuclear to cytoplasmic ratio indicating the presence of a CMC is less than 2.5. In some embodiments, the nuclear to cytoplasmic ratio indicating the presence of a CMC is less than 2.0. In some embodiments, the nuclear to cytoplasmic ratio indicating the presence of a CMC is less than 1.5. In some embodiments, the nuclear to cytoplasmic ratio indicating the presence of a CMC is less than 1.0.

In some embodiments, the methods of the disclosure include detection of high definition CMCs (HD-CMCs). HD-CMCs can be, in some embodiments, CSPG4 (+) with an SDOM of greater than or equal to 2, Hoechst stain (+), CD45 (-), and have a morphologically distinct feature from surrounding WBCs including having an intact nucleus with a nuclear to cytoplasmic ratio of less than 2.5. CSPG4 (+), Hoechst stain (+) and CD45 (-) intensities can be categorized as measurable features during HD-CTC enumeration as described herein and/or as described in Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CMC population, including a population that is heterogeneous as described herein.

While CMCs can be identified as being CSPG4 (+), Hoechst stain (+) and CD45 (-) cells, the methods of the invention can be practiced with any other biomarkers that one of skill in the art selects for generating CMC data and/or identifying CMCs. One skilled in the art knows how to select a morphological feature, biological molecule, or a fragment of a biological molecule, the change and/or the detection of which can be correlated with a CMC.

CMCs, which can be present as single cells or in clusters of CMCs, can be cells shed from solid melanoma tumors and be present in very low concentrations in the circulation of subjects. Accordingly, detection of CMCs in a blood sample can be referred to as rare event detection. CMCs can have an abundance of less than 1:1,000 in a blood cell population, *e.g*., an abundance of less than 1:5,000, 1:10,000, 1:30,000, 1:50:000, 1:100,000, 1:300,000, 1:500,000, or 1:1,000,000. In some embodiments, the CMC has an abundance of 1:50:000 to 1:100,000 in the cell population.

The samples of this disclosure may be obtained by any means, including, *e.g.*, by means of solid tissue biopsy or fluid biopsy (*see*, *e.g.,* Marrinucci D. et al., 2012, Phys. Biol. 9 016003). A blood sample may be extracted from any source known to include blood cells or components thereof, such as venous, arterial, peripheral, tissue, cord, and the like. The samples may be processed using well known and routine clinical methods (*e.g.*, procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

In some embodiments, the methods of this disclosure comprise an initial step of obtaining a WBC count for the blood sample. In certain embodiments, the WBC count may be obtained by using a HemoCue® WBC device (Hemocue, Ängelholm, Sweden). In some embodiments, the WBC count is used to determine the amount of blood required to plate a consistent loading volume of nucleated cells per slide and to calculate back the equivalent of CMCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e.g.*, in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells (*See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of the disclosure, including determining the presence or absence of one or more detectable agents in or on nucleated cells in the sample and/or assessing the morphology of the nucleated cells having the one or more detectable agents can be performed by automated fluorescent microscopy. For example, fluorescent scanning microscopy to detect immunofluorescent staining of nucleated cells in a blood sample has been described by Marrinucci D. et al., 2012, Phys. Biol. 9 016003, and can be used with the disclosed methods. However, a person skilled in the art will appreciate that a number of methods can be used to identify CMCs in a biological sample, including microscopy based approaches, mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunoprecipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will further appreciate that the presence or absence of biomarkers may be detected using any class of marker-specific binding reagents known in the art, including, *e.g.*, antibodies, aptamers, fusion proteins, such as fusion proteins including protein receptor or protein ligand components, or biomarker-specific small molecule binders. In some embodiments, the presence or absence of CSPGT4, PmeL17 or S100A1 is determined by an antibody.

The antibodies of this disclosure can bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability can also be used. The antibody can have a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

One or more detectable agents can be used in the methods described herein for direct or indirect detection of the biomarkers when identifying CMCs in the methods of the disclosure. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.*, fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (*e.g.*, green fluorescent protein (GFP), phycoerythrin, etc.), enzymes (*e.g.*, luciferase, horseradish peroxidase, alkaline phosphatase, etc.), nanoparticles, biotin, digoxigenin, metals, and the like.

For mass-sectrometry based analysis, differential tagging with isotopic reagents, *e.g.*, isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome can also be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

CMCs can be detected with any microscopic method known in the art. In some embodiments, the method is performed by fluorescent scanning microscopy. In certain embodiments the microscopic method provides high-resolution images of CMCs and their surrounding WBCs (*see, e.g.,* Marrinucci D. et al., 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, identifying CMCs includes detecting one or more biomarkers, for example, CSPG4, Pmel17, S100A1 or CD45. A biomarker is considered "present" in a cell if it is detectable above the background noise of the respective detection method used (*e.g.,* 2-fold, 3-fold, 5-fold, or 10-fold higher than the background; *e.g.,* 2σ or 3σ over background). In some embodiments, a biomarker is considered "absent" if it is not detectable above the background noise of the detection method used (*e.g.,* <1.5-fold or <2.0-fold higher than the background signal; *e.g.,* <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CMC-specific immunofluorescent markers, even though absent on WBCs are visible in the WBCs as background signals with fixed heights. Moreover, WBC-specific immunofluorescent markers that are absent on CMCs are visible in the CMCs as background signals with fixed heights. In some embodiments, a cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; *e.g.*, 2σ or 3σ over background). For example, a nucleated cell can be considered CD45 (+) if its fluorescent signal for CD45 is significantly higher than the background signal. A cell is considered negative for an immunofluorescent marker (*i.e.,* the marker is considered absent) if the cell's fluorescence signal for the respective marker is not significantly above the background signal (*e.g.,* <1.5-fold or <2.0-fold higher than the background signal; *e.g.,* <1.5σ or <2.0σ over background).

Typically, each microscopic field contains both CMCs and WBCs. In certain embodiments, the microscopic field shows at least 1, 5, 10, 20, 50, or 100 CMCs. In certain embodiments, the microscopic field shows at least 10, 25, 50, 100, 250, 500, or 1,000 fold more WBCs than CMCs. In certain embodiments, the microscopic field comprises one or more CMCs or CMC clusters surrounded by at least 10, 50, 100, 150, 200, 250, 500, 1,000 or more WBCs.

The methods for diagnosing include enumeration of CMCs that are present in the blood sample. In some embodiments, a positive diagnosis of metastatic melanoma comprises detection of at least 0.5 CMC/ml of blood, 1.0 CMC/mL of blood, 1.5 CMCs/mL of blood, 2.0 CMCs/mL of blood, 2.5 CMCs/mL of blood, 3.0 CMCs/mL of blood, 3.5 CMCs/mL of blood, 4.0 CMCs/mL of blood, 4.5 CMCs/mL of blood, 5.0 CMCs/mL of blood, 5.5 CMCs/mL of blood, 6.0 CMCs/mL of blood, 6.5 CMCs/mL of blood, 7.0 CMCs/mL of blood, 7.5 CMCs/mL of blood, 8.0 CMCs/mL of blood, 8.5 CMCs/mL of blood, 9.0 CMCs/mL of blood, 9.5 CMCs/mL of blood, 10 CMCs/mL of blood, 20 CMCs/mL of blood, 30 CMCs/mL of blood, 40 CMCs/mL of blood, 50 CMCs/mL of blood, 60 CMCs/mL of blood, 70 CMCs/mL of blood, 80 CMCs/mL of blood, 90 CMCs/mL of blood, 100 CMCs/mL of blood, 200 CMCs/mL, 300 CMCs/mL, 400 CMCs/mL, 500 CMCs/mL or more. In a particular embodiment, a positive diagnosis of metastatic melanoma includes detection of at least 0.5 CMC/mL of blood. In a particular embodiment, a positive diagnosis of metastatic melanoma includes detection of at least 1 CMC/mL of blood. In a particular embodiment, a positive diagnosis of metastatic melanoma includes detection of at least 2 CMC/mL of blood. In a particular embodiment, a positive diagnosis of metastatic melanoma includes detection of at least 5 CMC/mL of blood. In a particular embodiment, a positive diagnosis of metastatic melanoma includes detection of at least 10 CMC/mL of blood. In a particular embodiment, a positive diagnosis of metastatic melanoma includes detection of at least 20 CMC/mL of blood. In a particular embodiment, a positive diagnosis of metastatic melanoma includes detection of at least 50 CMC/mL of blood. In a particular embodiment, a positive diagnosis of metastatic melanoma includes detection of at least 100 CMC/mL of blood. In a particular embodiment, a positive diagnosis of metastatic melanoma includes detection of at least 500 CMC/mL of blood.

In some embodiments of the methods for diagnosing, the disclosed method can include detecting CMC clusters. In some embodiments, a positive diagnosis of metastatic melanoma includes detection of at least 0.1 CMC cluster/mL of blood, 0.2 CMC clusters/mL of blood, 0.3 CMC clusters/mL of blood, 0.4 CMC clusters/mL of blood, 0.5 CMC clusters/mL of blood, 0.6 CMC clusters/mL of blood, 0.7 CMC clusters/mL of blood, 0.8 CMC clusters/mL of blood, 0.9 CMC clusters/mL of blood, 1 CMC cluster/mL of blood, 2 CMC clusters/mL of blood, 3 CMC clusters/mL of blood, 4 CMC clusters/mL of blood, 5 CMC clusters/mL of blood, 6 CMC clusters/mL of blood, 7 CMC clusters/mL of blood, 8 CMC clusters/mL of blood, 9 CMC clusters/mL of blood, 10 clusters/mL or more. In a particular embodiment, a positive diagnosis of metastatic melanoma comprises detection of at least 0.1 CMC cluster/mL of blood. In a particular embodiment, a positive diagnosis of metastatic melanoma comprises detection of at least 0.5 CMC cluster/mL of blood. In a particular embodiment, a positive diagnosis of metastatic melanoma comprises detection of at least 1 CMC cluster/mL of blood. In a particular embodiment, a positive diagnosis of metastatic melanoma comprises detection of at least 2 CMC cluster/mL of blood. In a particular embodiment, a positive diagnosis of metastatic melanoma comprises detection of at least 10 CMC cluster/mL of blood.

In some embodiments, the method disclosed herein for diagnosing metastatic melanoma in a subject has a specificity of >60%, >70%, >80%, >90% or higher. In additional embodiments, the method for diagnosing metastatic melanoma in a subject has a specificity >60% at a classification threshold of 0.5 CMCs/mL of blood. In additional embodiments, the method for diagnosing metastatic melanoma in a subject has a specificity >70% at a classification threshold of 0.5 CMCs/mL of blood. In additional embodiments, the method for diagnosing metastatic melanoma in a subject has a specificity >80% at a classification threshold of 0.5 CMCs/mL of blood. In additional embodiments, the method for diagnosing metastatic melanoma in a subject has a specificity >90% at a classification threshold of 0.5 CMCs/mL of blood.

### EXAMPLES

### Example 1. Fluid Biopsy for Melanoma

This example describes a new high definition circulating tumor cell (HD-CTC) assay to identify CMCs in melanoma patients. Using the described HD-CTC assay, CMCs can be characterized and identified by a combination of detectable markers and morphological characteristics. The HD-CTC assay can quantify the small number of CMCs in the presence of a much larger number of white blood cells.

HD-CTC technology is distinct in that it does not rely on any protein-based enrichment strategy to detect CTCs, but uses multi-parametric computational analysis instead. All nucleated cells are retained and immunoflurescently labeled with tissue-specific monoclonal antibodies. Importantly, cells that do not meet specific selection criteria are not discarded. Therefore, each individual cell and its associated analysis data are catalogued in databases for subsequent re-analysis. Moreover, this strategy does not alter cell morphology allowing detailed pathologic analysis of the CTC population that is known to be very heterogeneous. This immunoenrichment-free strategy has been proven to yield high sensitivity and specificity for CTC detection in a variety of epithelial cancers, and results in substantially higher sensitivity than the current standard and FDA-approved CellSearch® methodology.

The biomarker selected for the detection of CMCs in the present example is specific for chondroitin sulfate proteoglycan 4 (CSPG4), also known as high molecular weight melanoma associated antigen (HMW-MAA) and melanoma chondroitin sulfate proteoglycan (MCSP). CSPG4 is reported to be highly expressed on melanoma cells in at least 80% of melanoma tumors and has limited distribution in other tissues (Campoli et al., Crit Rev Immunol. 2004;24(4):267-96). CSPG4 is continuously expressed throughout the course of the disease, and does not appear to be affected by the therapies used in melanoma (Yang et al., J Cell Biol. 2004 Jun 21;165(6):881-91). It has also been used to identify metastatic melanoma cells in sentinel lymph nodes by RT-PCR and immunohistochemistry (Goto et al., Clin Cancer Res Off J Am Assoc Cancer Res. 2008 Jun 1;14(11):3401-7). Additionally, soluble CSPG4 has been used to detect CMCs in serum samples from melanoma patients (Vergilis et al., J Invest Dermatol. 2005 Sep;125(3):526-31), and immunomagnetic enrichment of CMCs targeting CSPG4 has been shown (Ulmer et al., Clin Cancer Res Off J Am Assoc Cancer Res. 2004 Jan 15;10(2):531-7; Kitago et al., Clin Chem. 2009 Apr;55(4):757-64; Sakaizawa et al., Br J Cancer. 2012 Feb 28;106(5):939-46). A panel of seven high-affinity monoclonal antibodies (mAbs) raised against distinct and distant epitopes of CSPG4 are used. These mAbs provide reliable identification of different CMCs subpopulations based on CSPG4 protein expression, size and morphology and show the phenotypic heterogeneity of CMCs within and across melanoma patients.

### METHODS

### Melanoma cell lines and spiking experiments

The three cell lines used in this example represent the major stages of melanoma progression: radial growth phase (WM1789), vertical growth phase (WM278) and metastasis (WM1617). All three melanoma cell lines were purchased from the Wistar Institute Collection at the Coriel Institute for Medical Research, Camden, NJ, USA. The cells were maintained in Tu2% melanoma growth medium, consisting of four parts of MCDB153 (Sigma-Aldrich, Saint Louis, MO, USA) and one part of L-15 (Invitrogen, Carlsbard, CA, USA), supplemented with 1.68 mmol/L calcium chloride, 5 µg/ml insulin and 2% fetal bovine serum (Invitrogen, Carlsbard, CA, USA). The culture medium was changed every 2 days. To determine expression of markers, melanoma cells were spiked into peripheral blood mononuclear cells (PBMCs) in 1:100 ratio. To evaluate sensitivity, 0, 10, 50, 100 and 500 cells were spiked in three million PBMCs. To mimic patient samples, the melanoma cells, removed from the culture plate with 0.02% Versene (Lonza, Walkersvill, MD, USA) to preserve membranous expression of CSPG4, were added to the PBMC pellet obtained after red blood cell lysis. The assay was repeated three times to validate the reproducibility of the assay.

### CSPG4 monoclonal antibodies

The CSPG4-specific mAb 149.53, 763.74, TP61.52, VF1-TP41.2, VF4-TP108, VF4-TP109.2, VT80.12 were developed and characterized as previously described (Campoli et al., Crit Rev Immunol. 2004;24(4):267-96; Goto et al., Clin Cancer Res Off J Am Assoc Cancer Res. 2008 Jun 1;14(11):3401-7.21; Giacomini et al., J Immunol. 1985 Jul;135(1):696-702; and Temponi et al., Hybridoma 1989 Feb;8(1):85-95). The purity of mAb preparations was assessed by SDS-PAGE analysis, and the activity of mAb preparations was monitored by testing with CSPG4-bearing melanoma cells in a binding assay.

### HD-CTC assay for melanoma

For establishing the CSPG4 staining protocol, preparations of the three melanoma cell lines spiked with and without PBMCs from a normal donor *were used. To* optimize the staining procedure, *all relevant parameters of the protocol* were evaluated as follows: staining before and after *slide fr*eezing (see blood sample processing below); *cell fixation* was 20 minutes at room temperature with 2 % neutral buffered formalin solution before or after freezing; total antibody concentration used was 0.07, 0.15, 0.3, 0.6, 1.25, 2.5, 5, 7.5 and 12 µg/ml made in *10% goat serum; incubation* times for primary antibodies were 40 min, 2 hours and overnight; incubation time for secondary antibody was 40 min. The optimal CSPG4 staining (low background, membranous staining in melanoma cells and no staining in WBC) was determined to be as follows: After the slides were thawed and fixed with 2% neutral buffered formalin solution (100503, VWR, CA) non-specific binding sites were blocked with 10% goat serum (Millipore, CA). Slides were subsequently incubated with CSPG4-specific mAbs (5 µg/ml total concentration) and Alexa 647 preconjugated anti-CD45 antibody (MCA87A647, AbD serotec, USA) for 40 min at 37 °C. After a washing step with PBS, slides were incubated with Alexa Fluor 555 goat antimouse IgG1 antibody (A21127, Life technologies, USA) for 20 min at 37°C. Cells were counterstained with Hoechst 33258 (H1398, Life technologies, USA) for 30 min at room temperature and mounted with an aqueous mounting media. Slides of PBMCs with no added tumor cells served as a negative control. Further characterization of CSPG4 positive cells was performed using a mAb targeting HMB45 (clone gp-100, Dako, Denmark), a tissue-specific marker for melanocytes (Alexa Fluor 488 goat antimouse IgG1 antibody (A21121, Life technologies, USA)).

### Patient population and blood sample collection

All metastatic melanoma patients enrolled in this study were enrolled prospectively and signed consent forms for the use of blood samples and physical and medical records. All samples were de-identified after the blood was drawn. 8 ml of peripheral blood was collected from 40 metastatic melanoma patients in anti-coagulated Cyto-Chex® BCT tubes (Streck Innovations, Omaha, NE) according to institution specific IRB-approved protocols. Samples from non-local sites (Comprehensive Cancer Centers of Nevada, Las Vegas, NV) were shipped overnight so that the sample were received and processed within 24 h. Samples from local sites (Pacific Oncology and Hematology, Encinitas, CA) were held at room temperature for 24 h to mimic samples coming from non-local sites. Blood specimens were also drawn from 10 normal blood donors (NBDs) from The Scripps Research Institute's Normal Blood Donor Service. The analysis of the samples was conducted with no previous knowledge of patient's disease status. Clinical data including age, date of initial diagnosis, histology, LDH levels, performance status and BRAF status amongst others were collected retrospectively.

### Blood sample processing for HD-CMC enumeration and characterization

Whole blood specimens were prepared according the following method. Blood tubes were rocked for 5 min before a WBC count was performed using the Hemocue WBC system (HemoCue, Sweden). Based upon the WBC count, a volume of blood was subjected to an isotonic erythrocyte lysis with NH4C1 (ammonium chloride) buffer. After centrifugation, nucleated cells were re-suspended in PBS to a final concentration of 4 million cells per ml, plated as a monolayer on custom made glass slides and incubated for 40 min at 37°C. The glass slides are the same size as standard microscopy slides, but the slides have a proprietary coating that allows maximal retention of live cells. Each slide can hold approximately three million nucleated cells; thus the number of slides obtained depended on the patients' WBC count. After the incubation time, the slides are coated with 7% BSA for 5 min and finally dried on a heat block at 37°C for 12 min. The slides created for each patient are then stored at -80°C for future experiments. For HD-CMC detection in melanoma patients, four slides holding approximately twelve million cells were used as a test.

### Imaging and technical analysis

All nucleated cells in the specimen were imaged by a fluorescent automated microscope. Each slide was scanned entirely at 10X magnification and produced 2304 images per channel used. The obtained images were analyzed using custom computer algorithms and the resulting HD-CMC cell candidates were identified based upon numerous measures, including CSPG4 and CD45 intensity, Hoechst positivity, and nuclear and cellular morphology. Finally, the HD-CMC candidates were evaluated by direct review and classified as a HD-CMC or not based on immunophenotype and cell morphology. Other cells related to CMCs but lacking essential features of a tumor cell were also tracked and classified. CMC counts were reported per milliliter of blood (CTCs ml⁻¹). The value is calculated by counting the total number of nucleated cells on the glass slide used to isolate and detect CMCs and comparing it to the PBMC count in the patient's blood specimen. The ratio of counted nucleated cells over the PBMCs per milliliter in the blood specimen determined the volume of blood used per test (four slides). For this reason, fractional values of CMCs ml⁻¹ are possible.

### Re-location and Re-imaging

The HD-CMCs were relocated and reimaged using a macro written for ImagePro Plus (Media Cybernetics, Bethesda, MD). The images were taken at a fixed exposure intensity and gain at 40x magnification on a Nikon 80i (Melville, NY) epifluorescent microscope equipped with a QImaging Retiga EXi 12-bit monochrome CCD camera (QImaging, Surrey, BC, Canada).

### Statistical analysis

Relative CSPG4 values measured in cell lines were compared by one-way ANOVA with a Mann Whitney correction test using GraphPad Prism.

As CMC levels are not normally distributed, non-parametric tests were used. Means between two groups were compared using the Mann-Whitney test. One tailed P values of < 0.05 were considered significant.

### RESULTS

### CSPG4 protein expression, sensitivity and specificity on melanoma cells

The expression of CSPG4 protein was first assessed by immunofluorescence analysis with the following CSPG4-specific mAbs 149.53, 763.74, TP61.5, VF1-TP41.2, VF4-TP108, VF4-TP109.2, and VT80.12 to verify expression, sensitivity and specificity of each specific mAb on three melanoma cell lines under spiking conditions where melanoma cells were mixed with PBMCs from a normal donor control. CSPG4 protein expression was detected on the surface of all three melanoma cell lines (**FIG. 1A**). Each individual mAb recognizes different and distant epitopes of CSPG4. Expression of the targeted epitopes recognized by mAb VT801.2 and TP61.6 were more commonly expressed across cell lines, whereas those recognized by mAb 149.53 and TP41.2 more heterogeneously distributed (**FIG. 1B**). This heterogeneous expression of CSPG4 epitopes provided the rationale for the use of a combination of all 7 CSPG4-specific mAbs in this assay. The intensity of the staining provided by the combination of all 7 CSPG4-specific mAbs was higher than those obtained using individual mAbs at the same concentration. PBMCs from a normal blood donor were used to normalize signal intensities. Based on immunofluorescent parameters, an intact HD-CMC was defined as a cell that was: CSPG4 positive, CD45 negative, with an intact non-apoptotic nucleus by Hoechst imaging. Positivity for CSPG4 was defined as the fluorescent signal being at least 2 fold the background signal of surrounding PBMCs. Negativity of CD45 was defined as having signal below visual detection under the condition that 99% of all surrounding PBMCs were detectable globally.

### HD-CMC assay linearity by enumeration of melanoma cells spiked in PBMCs

To test assay linearity using the combination of all 7 CSPG4-specific mAbs, serial dilutions of melanoma cells (0, 10, 50, 100 and 500) were spiked into approximately 3 x 10⁶ PBMC from a normal blood donor in triplicates and processed according to the HD-CMC assay. As displayed in **FIG. 2****,** the number of WM1617, WM278 and WM1789 cells detected is plotted against expected cells. A percentage of detection of 97, 98.3 and 97.3 with a linear detection coefficient of 0.99, 0.99 and 0.97 was obtained using WM1617 **(****FIG. 2A****),** WM278 **(****FIG. 2B****)** and WM1789 **(****FIG. 2C****)** cell lines, respectively.

### Assay specificity by comparison of normal blood donors and melanoma patients' samples using the HD-CMC definition

To assess the specificity of the assay, 10 blood samples from normal donors and 40 from melanoma patients were compared **(****FIG. 3****).**

Samples from normal donors were evaluated as a control population consisting of 6 females and 4 males with an age range of 40 to 79 years. A total of 105 candidate cells were found. Upon a post - classification analysis, 48% of cells (55 cells) did not meet one of the inclusion criteria by having a CSPG4 signal below the cutoff and were easily excluded. Explicit review of the rest of cells (52% of total) revealed a similar pattern, in that they were near to the lower limit for inclusion by one or more criteria. In general, these cells were CD45 negative, had an intact nucleus, and had a CSPG4 signal intensity up to 7.8-fold brighter than the surrounding PBMCs. However, this signal did not follow the CSPG4 pattern characterized by a signal distribution on the cell membrane, and visually did not appear to be significantly brighter than surrounding PBMCs by single channel fluorescent analysis. In addition to that, analysis performed by a histopathologist confirmed that they were morphologically different than surrounding PBMCs with different shapes and enlarged nuclei but they did not have a morphology compatible with a malignant phenotype based on by criteria used in standard diagnostic cytopathology such us enlarged size, architectural organization of nucleus and cytoplasm, cytoplasmic shape, and nuclear shape.

Samples from 40 melanoma patients were also evaluated in parallel as a test population consisting in 15 females and 25 males with an age range of 45 to 91. In this patient group, 740 candidate cells were identified. Seventeen percent of them (124 cells) had a CSPG4 signal below the cutoff and were excluded. The remaining eighty three percent (616 cells) fulfill strictly all the inclusion criteria. On average, these cells had a mean CSPG4 intensity of 31.8, and a standard deviation of 49.3. 68% (415 cells) had a visually bright relative CSPG4 signal ranging from 8 to 389 and 32% (198 cells) were near to the lower limit of CSPG4 signal, being just between 2- and 8-fold brighter than the surrounding PBMCs. Upon thorough post-classification review of these margin cells, a morphologically heterogeneous population of HD-CTCs was observed within and across the patients. HD-CTCs had various cellular and nuclear shapes and sizes, and different CSPG4 patterns. Since cells in this range of CSPG4 intensity were also detected in samples from normal blood donors, we assumed that normal blood cells may also exist among cells that populate this group (CSPG4 low, CD45 negative, intact nucleus) in melanoma patients. Excluding this group of cells by setting the CSPG4 intensity cutoff at 8 would increase the specificity of the assay but would decrease the sensitivity leading in false negative results. On the other hand, including this group by keeping the cutoff at 2 would lead to false positive results. Image analyses of physical characteristics of these cells were made to determine quantitative differences that may functionally contribute to improve inclusion criteria for a cell to be defined as a HD-CMC.

### Tailoring HD-CMC definition

The HD-CTC platform allows for simultaneous cytomorphologic review of fluorescent images for the individual channels with cell-by-cell automatic annotation of data regarding size and fluorescent intensity of objects. Based on immunofluorescent parameters, an intact HD-CMC was defined as a cell that was: CSPG4 positive (at least 2-fold brighter than surrounding PBMCs), CD45 negative, with an intact non-apoptotic nucleus by Hoechst imaging. In addition to these characteristics and based on cytomorphologic parameters, HD-CMCs must be distinct from normal PBMCs, and must have a morphology that is compatible with a malignant phenotype by criteria used in standard diagnostic cytopathology such us enlarged size, architectural organization of nucleus and cytoplasm, cytoplasmic shape, and nuclear shape. Upon review and quantification of physical parameters of all cells detected in both normal controls and melanoma patients, relative nuclear size was the most informative parameter among others. It was calculated as the ratio of individual nuclear sizes of candidate cell (in pixels) and the mean nuclear size of surrounding PBMCs. Seventy percent of cells (516) detected in melanoma patients had a relative nuclear size smaller than 2.5 with an average of 1.5. The remaining 30% (224 cells) had a relative nuclear size up to 13-fold larger than the surrounding PBMCs with an average close to 5. The results obtained from normal donor samples showed that 100% of cells had a nuclear size of at least 2.5-fold larger than surrounding PBMCs, with an average of 5. Based on this evaluation we defined a new exclusion criterion for those cells near to the lower limit of CSPG4 intensity. Thus, cells from patients that were CD45 negative, CSPG4 low, had an intact nucleus and had a relative nuclear size of 2.5 or larger were excluded. This population represented 54% of cells (108) initially included in the low CSPG4 group based on immunofluorescent parameters. After complete immunocytochemical detection and cytomorphical analysis of the HD-CMCs candidates, 84.9 % of them were identified as HD-CMCs (CSPG4 mean 42.7 ± 54.29).

### Patient demographics and HD-CMC data

Between November 2010 and August 2013, 40 patients were recruited in this study and their clinical data are shown in detail in **Table 1**. The 40 patients comprised 25 males and 15 females, with a median age of 55.5 years (range 45-91 years). All patients have metastatic disease, as determine by radiological and clinical criteria at the time of blood draw. Metastatic sites included brain (12), lung (13), liver (8), bone (7), adrenal gland (4), subcutaneous (8) and skin (9). Thirty patients had stage IV and ten patients had stage IIIC. BRAF mutational status was assessed in 25 of patients and 11 of them had the mutation V600E detected. Twenty-one patients with stage IV and one with stage IIIC had died by the time of analysis, 3 were in complete remission, 13 were alive with disease, 2 were in progression and one was lost to follow-up. The follow-up period ranged from 0.08 to 22 months.

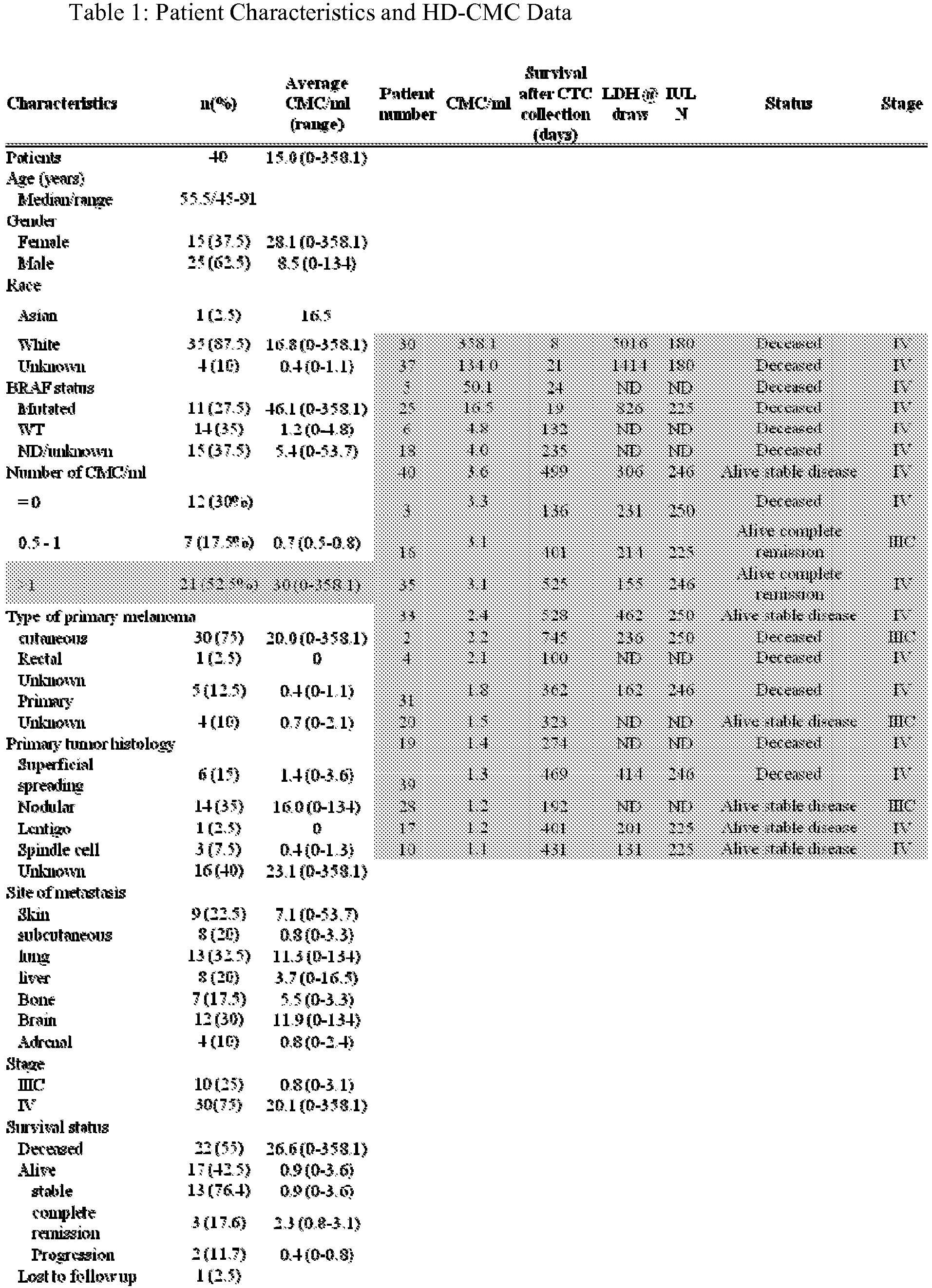

### Prevalence of HD-CMC in metastatic melanoma patients

Following the strict inclusion criteria, we detected 1 and 241 HD-CMC in 28 (70%) of 40 metastatic melanoma patients. The number of HD-CMCs ranged between 0.5 and 358.1/mL (mean 15.1), while no HD-CMCs were detected in the blood of normal blood donors **(****FIG. 4A****).** Twenty one of the positive patients (75%) had ≥ 1 HD-CTCs ml-1; 14 (50%) ≥ 2 HD-CTCs ml-1, 4 (14.3%) ≥ 10 HD-CTCs ml-1 and 2 (7%) ≥ 100 HD-CTCs ml-1. The CSPG4 signal intensity within the HD-CMC population varied within and across patients **(****FIG. 4B****).** Five patients (2, 3, 9, 31 and 38) accounted only with CSPG4 bright HD-CMCs, 14 patients (4, 8, 11, 13-15, 17-18, 21, 24-25, 28, 35 and 39) had only CSPG4 dim HD-CMCs, and 9 patients (5, 6, 10, 19, 20, 30, 33, 37 and 40) had both CSPG4 bright and CSPG4 low HD-CMCs. In the latter group, 4 patients (5, 30, 33 and 37) had mostly GSPG4 bright cells with a percentage ranging from 67 to 95%, 2 patients (6 and 10) had equal numbers and 3 patients (19, 20 and 40) had mostly CSPG4 low HD-CMCs ranging from 66% to 87.5%. **FIG. 4D** shows the cytomorphology and immunophenotype of 2 representative HD-CMCs from the three melanoma patients (patient 5, 30 and 37). To evaluate cytomorphologic heterogeneity on HD-CMCs, we analyzed the cells found in the two patients (30 and 37) with more than 100 HD-CTCs ml-1 to deliver meaningful results **(****FIG. 4C, 4D and 4E****)**. HD-CMC shapes varied within and across patients. Specially, most of the cells from patient 30 were high pleomorphic in shape and presented polygonal nuclei **(****FIG. 4C****).** Roundness mean was 1.01 in average for patient's PBMC, and 1.1 and 1.04 for HD-CMCs in patient 30 and 37 respectively, indicating that HD-CMCs were slightly oblong in shape compared to the surrounding PBMCs **(****FIG. 4D****).** Area plots indicate that HD-CMCs in melanoma patient 30 and 37 were significantly larger than their corresponding PBMCs (Mean area: 2x10⁻³ vs. 1x10⁻³, P<0.0001 and 1.7x10⁻³ vs. 1x10⁻³, P<0.0001, respectively) **(****FIG. 4D****).** In most of patients, only single cells were detected, except for 4 patients containing from two-cell to four-cell clusters.

### Characterization of HD-CMCs with HMB-45

Expression of HMB45 in combination with CSPG4 was first evaluated on melanoma cell lines and was detected in the cytoplasm of WM1789 and WM1617 cells lines, while no detection was observed in WM278 (data not shown). Same analysis was performed in the cohort of 40 metastatic melanoma patients (1 slide each). Cells were first selected based on the previously established inclusion criteria for an object to be selected as a HD-CMC. All HD-CMCs detected were then exhaustively analyzed and classified as either HMB45 positive (HMB45+) or negative (HMB45 -). **FIG. 5A** shows the distribution of 124 HD-CMCs found in 40 melanoma patients that were either CSPG4⁺/HMB-45⁻ (left) or CSPG4⁺/HMB-45⁺ (right). Sixty-one of 124 cells (49%) were CSPG4⁺/HMB-45⁻, while the remaining 68 cells (51%) were CSPG4⁺/HMB-45⁺. To evaluate signal heterogeneity of HD-CMCs within and across patients, we analyzed cells found in the two melanoma patients (30 and 37) who accounted with more than 100 HD-CTCs ml-1 each to obtain significant results. In patient number 30, 58 cells were reviewed. In general, HD-CMCs from this patient had a high relative CSPG4 intensity (mean 62.9). Only 33 of them (57%) were positive for both CSPG4 and HMB-45. Mean relative HMB-45 intensity of double positive cells was 20.7. In patient number 37, 37 cells were evaluated. In this case, twenty -four cells (68%) were positive for both CSPG4 and HMB-45. Mean relative CSPG4 intensity was 16.3, while mean relative HMB-45 intensity was 34.6 in the positive setting. **FIG. 5B** shows the immunologic heterogeneity observed between HD-CMCs within and across these two patients. Despite the low sensitivity of the HMB-45 marker observed in our assay, its high specificity supported the inclusion of those HD-CMCs accounting with low CSPG4 signal intensity, especially in patient number 37 where 26% of HD-CMCs had a relative CSPG4 intensity signal close to the lower limit cutoff.

### CTC levels and clinical outcome of melanoma patients

One aspect of this study was to adapt HD-CTC technology for the detection of CMCs. The number of patients in this study (n = 40) was thus not powered for survival analysis nor was the sampling of blood controlled for line of therapy. Nevertheless, there was an association between the number of CMC per ml of blood and the short survival observed in these patients. A receiver operating characteristic (ROC) curve was constructed using the results from melanoma patients (n = 39). A value of 10.7 HD-CTCs ml-1 was determined from this cohort data. OS time for those patients with < 10.7 HD-CTCs ml-1 was 315.9 days and was significantly longer than the median OS time for those patients with ≥ 10.7 CTCs ml-1, 18 days **(****FIG. 6****).**

In summary, the data shown herein, for the first time, demonstrates that CMCs are identified by the HD-CTC technology in 70% of metastatic melanoma patients and that CMCs constitute a very heterogeneous population in terms of CSPG4 expression and cell morphology within and across melanoma patients. The enrichment-free methods described enable direct immunocytochemical detection of CMCs using a panel of 7 CSPG4 mAbs that recognizes distinct and distant epitopes of CSPG4. Additionally, for the first time, expression of CSPG4 protein has been shown in WM1617, WM278 and WM1789 melanoma cell lines. Because CSPG4 epitope expression is unknown in CMCs and known to be heterogeneous in melanoma cell lines, use of a panel of CSPG4-specific mAbs for CMCs detection in melanoma patients can be an efficient strategy to detect CMCs. Additionally, use of a combination of CSPG4-specific mAbs markedly increased the intensity of the staining of melanoma cell lines. We also found that immunocytochemical characterization with HMB-45 could aid in the identification of HD-CMCs with low CSPG4 signal. As for the morphology of CMCs, in general, most had an enlarged size and were morphologically different than surrounding PBMCs. CMCs were also morphologically different within and across patients going from epitheliod cells with oval nuclei to polygonal cells with polarized nuclei. However, despite these cells displaying a wide range of CSPG4 intensities, the majority of the cells had a similar relative nuclear size, averaging 1.3 times larger than surrounding PBMCs. Interestingly, a subset of cells with low CSPG4 intensity had a range of nuclear sizes and were, on average, five-times larger than PBMCs. Similar large cells with low CSPG4 signal were also found in some NBD. Nevertheless, we were able to define strict inclusion criteria to safely exclude this borderline cells that were eventually recognized by a histopathologist as enlarged hematopoietic cells. Lastly, we observed that patients with more than 10.6 CMCs per ml of blood expired within 30 days of blood sample collection.

### Example 2. DNA Copy Number Variation Analysis of CMCs

This example describes the DNA analysis of single CMCs that were isolated from two melanoma patients identified in Example 1.

CMC extraction and copy number variation (CNV) analysis were conducted by relocating CMCs on the glass slide and reimaging at 40× for detailed cytomorphometric analysis as previously described in Marrinucci D. et al., 2012, Phys. Biol. 9 016003. CMCs were then captured by micromanipulation and whole genome amplification and CNV analysis were performed as previously described in Dago E. et al., 2014, PLoS ONE 9 e10177.

DNA CNVs were assessed in single CMCs, WBCs and 'excluded candidate cells' isolated from melanoma patient #30 (40 cells) and #37 (23 cells) (**FIGs. 7A-7F**). Chromosomal alterations were found in 100% of the CMCs analyzed. A unique clonal population (38 CMCs) in patient #30 and two clonal populations (18 CMCs) in patient #37 were observed. Chromosomal gains and deletions of chr5, 7, 9, 10, 12, 17, and 19 were detected in both patients.

Candidate genes encoding components of commonly altered pathways in melanoma were located at these amplified/deleted areas. For example, the amplification of mixed-lineage leukemia 3 (MLL3) in chromosome 7, an important histone regulator gene, and the loss of cyclin-dependent kinase inhibitor 2A (CDKN2A), a tumor suppressor gene that regulates the pRB and p53 pathways (Flores J F et al., 1996, Cancer Res. 56: 5023-32; and Hodis E et al., 2012, Cell 150: 251-63), were found to be present in both patients, along with an increase of a segment on chromosome 5p containing telomerase reverse transcriptase (TERT) locus, which encodes the catalytic protein subunit of the telomerase (Hodis E *et al., supra*). The loss of phosphatase and tensin homolog (PTEN), responsible for the negative regulation of the PI3K/AKT pathway (Paraiso K H T et al., 2011, Cancer Res. 71: 2750-60) was found only in patient #30. In patient #37, two CMC populations (clone A and B) were identified (**FIG. 7B**). Mouse double minute 2 homolog (MDM2), an important negative regulator of the p53 tumor suppressor (Muthusamy V et al., 2006, Genes. Chromosomes Cancer 45: 447-54), was amplified in all CMCs from both clones and have more than 20 copies each. Amplification of BRAF (Shi H et al., 2012, Nat. Commun. 3: 724), which regulates the MAPK signaling pathway, was identified in all CMCs from clone A. Kirsten rat sarcoma viral oncogene homolog (KRAS), involved primarily in regulating cell division, was only amplified in clone B. No chromosomal alterations were detected in the WBCs or 'excluded candidate cells' (data not shown). Thus, the above analysis of single CMCs indentified deletions of CDKN2A and PTEN, and amplification(s) of TERT, MLL3, BRAF, KRAS and MDM2.

The above results also showed a heterogeneous BRAF status between CMCs and matched tumor tissues as well as within the CMC population in individual patients. This suggests that the complex genomic diversity of melanoma is also illustrated in the CMC population.

Limited heterogeneity of CMCs in terms of chromosomal CNVs was also found when hierarchical cluster analysis was performed, consistent with previous reports. However, mutational analysis was not performed and it is possible that CMCs carry private mutations.

One important result associated with the genomic profiling shown above is the identification of novel altered chromosomal regions in CMCs in addition to markers of clinical significance known in melanoma. A complete deletion of CDKN2A together with the amplification of MDM2 in patient #37 suggest that the p53 pathway is inactivated (Zhang Y et al., 1998, Cell 92: 725-34) in this CMC population. Consistent with this, mutation analysis of cutaneous melanoma sequencing data showed that MDM2 amplification (4%) and CDKN2A deletion or mutation (42%) occur in around 55% of melanoma cases. Recent studies have supported the ability to restore the apoptotic function of p53 as a parallel therapeutic strategy alongside BRAFV600E inhibition in the treatment of melanoma (Lu M et al., 2013, Cancer Cell 23: 618-33; and Lu M et al., 2014, FEBS Lett. 558: 2616-21). Moreover, PTEN deletion in all CMCs from patient #30 and BRAF amplification in the CMCs populating clone A in patient #37 have been described as two distinct mechanisms of drug resistance after BRAF inhibitor therapy (Nazarian R et al., 2010, Nature: 468 973-7) (Shi H et al., 2012, Nat. Commun. 3: 724) and could, in part, explain why those patients progressed. Importantly, eight novel chromosomal amplifications are shown in chr12, 17 and 19 including cancer genes such as AKT2, PIK3C2 and BRIP1.

In light of the above results, screening for targetable genomic alternations at the single cell level can identify subpopulations of patients who will benefit from molecularly targeted therapies and allow their monitoring in real time.

## Claims

1. A method for identifying circulating melanoma cells (CMCs) in a biological sample comprising:
(a) contacting a biological sample of non-enriched blood with one or more detectable agents, wherein at least one of said one or more detectable agents is specific for a biomarker of CMCs, wherein the non-enriched sample is a sample not enriched for any specific population or subpopulation of nucleated cells;
(b) determining the presence or absence of said one or more detectable agents in or on nucleated cells in the sample; and
(c) assessing the morphology of the nucleated cells having said one or more detectable agents,
wherein the CMCs are identified based on a combination of the presence or absence of said one or more detectable agents and morphological characteristics of the nucleated cells.

2. A method for diagnosing metastatic melanoma comprising:
(a) contacting a biological sample of non-enriched blood with one or more detectable agents, wherein said sample was obtained from a subject suspected of having metastatic melanoma or diagnosed with having melanoma, wherein at least one of said one or more detectable agents is specific for a biomarker of circulating melanoma cells (CMCs), wherein the non-enriched sample is a sample not enriched for any specific population or subpopulation of nucleated cells;
(b) determining the presence or absence of said one or more detectable agents in or on nucleated cells present in the sample;
(c) assessing the morphology of the nucleated cells having said one or more detectable agents; and
(d) identifying the presence of CMCs in the sample based on a combination of the presence or absence of said one or more detectable agents and morphological characteristics of the nucleated cells,
wherein the subject is diagnosed with metastatic melanoma when a predetermined number of CMCs is present in the sample.

3. The method of claim 1 or 2, wherein said one or more detectable agents comprise a immunofluorescent marker.

4. The method of claim 3, wherein said immunofluorescent marker is an antibody or functional fragment thereof that specifically binds to chondroitin sulfate proteoglycan 4 (CSPG4) or premelanosome protein (Pmel17), or S100 calcium-binding protein A1 (S100A1);
optionally wherein said antibody is monoclonal.

5. The method of any one of claims 1-4 wherein said one or more detectable agents comprise two, three, four, five, six, seven or more immunofluorescent markers.

6. The method of any one of claims 1-5, wherein said one or more detectable agents comprise a nucleic acid specific stain;
optionally wherein said nucleic acid specific stain is a Hoechst stain.

7. The method of any one of claims 1-6, wherein said one or more detectable agents comprise an immunofluorescent marker for white blood cells (WBCs);
optionally wherein said immunofluorescent marker for WBCs is an antibody specific for cluster of differentiation 45 (CD45).

8. The method of any one of claims 1-7, wherein step (b) and/or (c) are performed by automated fluorescent microscopy.

9. The method of any one of claims 1-8, wherein said determining the presence or absence of said one or more detectable agents comprises comparing distinct immunofluorescent staining of CMCs with distinct immunofluorescent staining of white blood cells (WBCs).

10. The method of claim 9, wherein said immunofluorescent staining of CMCs is positive for an antibody or functional fragment thereof that specifically binds to CSPG4 and is detectable at a standard deviation of the mean (SDOM) of greater than or equal to 2; optionally wherein said immunofluorescent staining of CMCs is negative for an antibody or functional fragment thereof that specifically binds to CD45;
optionally wherein said immunofluorescent staining of CMCs is positive for Hoeschst staining.

11. The method of any one of claims 1-10, wherein said morphological assessment comprises comparing the morphological characteristics of CMCs with the morphological characteristics of surrounding white blood cells (WBCs);
optionally wherein said morphological characteristics comprise nucleus size, nucleus shape, cell size, cell shape or nuclear to cytoplasmic ratio;
optionally wherein a nuclear to cytoplasmic ratio of less than 2.5 indicates the presence of a CMC.

12. The method of any one of claims 1-11, further comprising obtaining a white blood cell (WBC) count for the sample.

13. The method of any one of claims 1-12, further comprising lysing erythrocytes in the sample.

14. The method of any one of claims 1-13, further comprising depositing nucleated cells from the sample as a monolayer on a glass slide;
optionally further comprising depositing about 3 million cells from the sample onto said glass slide.

15. The method of any one of claims 2-14, wherein said predetermined number of CMCs is at least 0.5, 1.0, 1.5, 2.0, 2.5, 3.0, 4.0, 5.0, 10, 20, 50, 100, 200, 300, 400 or 500 CMCs per ml of sample.

## Patentansprüche

1. Verfahren zum Identifizieren zirkulierender Melanomzellen (CMCs) in einer biologischen Probe, umfassend:
a) Kontaktieren einer biologischen Probe von nicht angereichertem Blut mit einem oder mehreren detektierbaren Mitteln, wobei mindestens eines des einen oder der mehreren detektierbaren Mittel für einen Biomarker von CMCs spezifisch ist, wobei die nicht angereicherte Probe eine Probe ist, die nicht mit einer spezifischen Population oder Subpopulation kernhaltiger Zellen angereichert ist;
b) Bestimmen der Gegenwart oder Abwesenheit des einen oder der mehreren detektierbaren Mittel in oder auf kernhaltigen Zellen in der Probe; und
c) Beurteilen der Morphologie der kernhaltigen Zellen, die das eine oder die mehreren detektierbaren Mittel aufweisen,
wobei die CMCs basierend auf einer Kombination der Anwesenheit oder Abwesenheit der einen oder mehreren detektierbaren Mittel und morphologischen Eigenschaften der kernhaltigen Zellen identifiziert sind.

2. Verfahren zum Diagnostizieren von metastatischem Melanom, umfassend:
a) Kontaktieren einer biologischen Probe von nicht angereichertem Blut mit einem oder mehreren detektierbaren Mitteln, wobei die Probe von einem Subjekt erhalten wurde, das unter Verdacht steht, metastatisches Melanom aufzuweisen, oder mit Melanom diagnostiziert wurde, wobei mindestens eines des einen oder mehreren detektierbaren Mittel für einen Biomarker von zirkulierenden Melanomzellen (CMCs) spezifisch ist, wobei die nicht angereicherte Probe eine Probe ist, die nicht mit einer spezifischen Population oder Subpopulation kernhaltiger Zellen angereichert ist;
b) Bestimmen der Anwesenheit oder Abwesenheit des einen oder der mehreren detektierbaren Mittel in oder auf kernhaltigen Zellen, die in der Probe vorliegen;
c) Beurteilen der Morphologie der kernhaltigen Zellen, die das eine oder die mehreren detektierbaren Mittel aufweisen; und
d) Identifizieren der Anwesenheit von CMCs in der Probe, basierend auf einer Kombination der Anwesenheit oder Abwesenheit des einen oder der mehreren detektierbaren Mittel und morphologischen Eigenschaften der kernhaltigen Zellen,
wobei das Subjekt mit metastatischem Melanom diagnostiziert ist, wenn eine vorgegebene Anzahl von CMCs in der Probe vorliegt.

3. Verfahren nach Anspruch 1 oder 2, wobei das eine oder die mehreren detektierbaren Mittel einen Immunfluoreszenzmarker umfassen.

4. Verfahren nach Anspruch 3, wobei der Immunfluoreszenzmarker ein Antikörper oder funktionales Fragment davon, das sich spezifisch an Chondroitinsulfatproteoglycan-4 (CSPDG4) oder Premelanosomprotein (Pmel17) oder S100-kalziumbindendes Protein A1 (S100A1) bindet, ist;
wobei optional der Antikörper monoklonal ist.

5. Verfahren nach einem der Ansprüche 1-4, wobei das eine oder die mehreren detektierbaren Mittel zwei, drei, vier, fünf, sechs, sieben oder mehr Immunfluoreszenzmarker umfassen.

6. Verfahren nach einem der Ansprüche 1-5, wobei das eine oder die mehreren detektierbaren Mittel einen Nukleinsäure-spezifischen Farbstoff umfassen;
wobei optional der Nukleinsäure-spezifische Farbstoff ein Hoechst-Farbstoff ist.

7. Verfahren nach einem der Ansprüche 1-6, wobei das eine oder die mehreren detektierbaren Mittel einen Immunfluoreszenzmarker für weiße Blutzellen (WBCs) umfassen;
wobei optional der Immunfluoreszenzmarker für WBCs ein Antikörper ist, der für Cluster of Differentiation 45 (CD45) spezifisch ist.

8. Verfahren nach einem der Ansprüche 1-7, wobei Schritt b) und/oder c) durch automatische Fluoreszenzmikroskopie durchgeführt sind.

9. Verfahren nach einem der Ansprüche 1-8, wobei das Bestimmen der Anwesenheit oder Abwesenheit des einen oder der mehreren detektierbaren Mittel das Vergleichen speziefischer Immunfluoreszenzfärbung von CMCs mit speziefischer Immunfluoreszenzfärbung von weißen Blutzellen (WBCs) umfasst.

10. Verfahren nach Anspruch 9, wobei die Immunfluoreszenzfärbung von CMCs für einen Antikörper oder ein funktionales Fragment davon, das spezifisch an CSPG4 bindet, positiv ist und bei einer Standardabweichung des Mittelwerts (SDOM) von mehr als oder gleich 2 detektierbar ist;
wobei optional die Immunfluoreszenzfärbung von CMCs für einen Antikörper oder ein funktionales Fragment davon negativ ist, der bzw. das spezifisch an CD45 bindet;
wobei optional die Immunfluoreszenzfärbung von CMCs für Hoechst-Farbstoff positiv ist.

11. Verfahren nach einem der Ansprüche 1-10, wobei die morphologische Beurteilung das Vergleichen der morphologischen Eigenschaften von CMCs mit den morphologischen Eigenschaften von umliegenden weißen Blutzellen (WBCs) umfasst;
wobei optional die morphologischen Eigenschaften Kerngröße, Kernform, Zellgröße, Zellform oder Kern-zu-Zytoplasma-Verhältnis umfassen;
wobei optional ein Kern-zu-Zytoplasma-Verhältnis von weniger als 2,5 die Anwesenheit einer CMC anzeigt.

12. Verfahren nach einem der Ansprüche 1-11, weiter umfassend das Erhalten einer Anzahl von weißen Blutzellen (WBC) in der Probe.

13. Verfahre nach einem der Ansprüche 1-12, weiter umfassend das Lysieren von Erythrozyten in der Probe.

14. Verfahren nach einem der Ansprüche 1-13, weiter umfassend das Deponieren kernhaltiger Zellen von der Probe als eine Monoschicht auf einer Glasträgerplatte;
optional weiter umfassend das Deponieren von etwa 3 Millionen Zellen von der Probe auf die Glasträgerplatte.

15. Verfahren nach einem der Ansprüche 2-14, wobei die vorgegebene Anzahl von CMCs mindestens 0,5, 1,0, 1,5, 2,0, 2,5, 3,0, 4,0, 5,0, 10, 20, 50, 100, 200, 300, 400 oder 500 CMCs pro ml Probe ist.

## Revendications

1. Procédé d'identification de cellules de mélanome circulantes (CMC) dans un échantillon biologique comprenant :
(a) la mise en contact d'un échantillon biologique de sang non enrichi avec un ou plusieurs agents détectables, dans lequel au moins l'un desdits un ou plusieurs agents détectables est spécifique d'un biomarqueur des CMC, dans lequel l'échantillon non enrichi est un échantillon non enrichi pour n'importe quelle population ou sous-population spécifique de cellules nucléées ;
(b) la détermination de la présence ou de l'absence desdits un ou plusieurs agents détectables dans ou sur les cellules nucléées dans l'échantillon ; et
(c) l'évaluation de la morphologie des cellules nucléées présentant lesdits un ou plusieurs agents détectables,
dans lequel les CMC sont identifiées sur la base d'une combinaison de la présence ou de l'absence desdits un ou plusieurs agents détectables et des caractéristiques morphologiques des cellules nucléées.

2. Procédé de diagnostic d'un mélanome métastatique comprenant :
(a) la mise en contact d'un échantillon biologique de sang non enrichi avec un ou plusieurs agents détectables, dans lequel ledit échantillon a été obtenu à partir d'un sujet suspecté d'avoir un mélanome métastatique ou diagnostiqué avec un mélanome, dans lequel au moins l'un desdits un ou plusieurs agents détectables est spécifique d'un biomarqueur de cellules de mélanome circulantes (CMC), dans lequel l'échantillon non enrichi est un échantillon non enrichi pour n'importe quelle population ou sous-population spécifique de cellules nucléées ;
(b) la détermination de la présence ou de l'absence desdits un ou plusieurs agents détectables dans ou sur les cellules nucléées présentes dans l'échantillon ;
(c) l'évaluation de la morphologie des cellules nucléées présentant lesdits un ou plusieurs agents détectables ; et
(d) l'identification de la présence de CMC dans l'échantillon sur la base d'une combinaison de la présence ou de l'absence desdits un ou plusieurs agents détectables et des caractéristiques morphologiques des cellules nucléées,
dans lequel le sujet est diagnostiqué avec un mélanome métastatique quand un nombre prédéterminé de CMC est présent dans l'échantillon.

3. Procédé selon la revendication 1 ou 2, dans lequel lesdits un ou plusieurs agents détectables comprennent un marqueur immunofluorescent.

4. Procédé selon la revendication 3, dans lequel ledit marqueur immunofluorescent est un anticorps ou un fragment fonctionnel de celui-ci qui se lie spécifiquement au chondroïtine sulfate protéoglycane 4 (CSPG4) ou à la protéine de prémélanosome (Pmel17), ou à la protéine A1 de liaison au calcium S100 (S100A1) ;
facultativement dans lequel ledit anticorps est monoclonal.

5. Procédé selon l'une quelconque des revendications 1 à 4, dans lequel lesdits un ou plusieurs agents détectables comprennent deux, trois, quatre, cinq, six, sept ou plus de sept marqueurs immunofluorescents.

6. Procédé selon l'une quelconque des revendications 1 à 5, dans lequel lesdits un ou plusieurs agents détectables comprennent un colorant spécifique d'acide nucléique ;
facultativement dans lequel ledit colorant spécifique d'acide nucléique est un colorant de Hoechst.

7. Procédé selon l'une quelconque des revendications 1 à 6, dans lequel lesdits un ou plusieurs agents détectables comprennent un marqueur immunofluorescent pour les globules blancs (WBC) ;
facultativement dans lequel ledit marqueur immunofluorescent pour les WBC est un anticorps spécifique de la classe de différenciation 45 (CD45).

8. Procédé selon l'une quelconque des revendications 1 à 7, dans lequel les étapes (b) et/ou (c) sont effectuées par microscopie de fluorescente automatisée.

9. Procédé selon l'une quelconque des revendications 1 à 8, dans lequel ladite détermination de la présence ou de l'absence desdits un ou plusieurs agents détectables comprend la comparaison d'une coloration immunofluorescente distincte des CMC avec une coloration immunofluorescente distincte des globules blancs (WBC).

10. Procédé selon la revendication 9, dans lequel ladite coloration immunofluorescente des CMC est positive pour un anticorps ou un fragment fonctionnel de celui-ci qui se lie spécifiquement à CSPG4 et est détectable à un écart-type de la moyenne (SDOM) supérieur ou égal à 2 ;
facultativement dans lequel ladite coloration immunofluorescente des CMC est négative pour un anticorps ou un fragment fonctionnel de celui-ci qui se lie spécifiquement à CD45 ;
facultativement dans lequel ladite coloration immunofluorescente des CMC est positive pour la coloration de Hoeschst.

11. Procédé selon l'une quelconque des revendications 1 à 10, dans lequel ladite évaluation morphologique comprend la comparaison des caractéristiques morphologiques des CMC avec les caractéristiques morphologiques des globules blancs environnants (WBC) ;
facultativement dans lequel lesdites caractéristiques morphologiques comprennent la taille du noyau, la forme du noyau, la taille de la cellule, la forme de la cellule ou le rapport nucléaire sur cytoplasmique ;
facultativement dans lequel un rapport nucléaire sur cytoplasmique inférieur à 2,5 indique la présence d'une CMC.

12. Procédé selon l'une quelconque des revendications 1 à 11, comprenant en outre l'obtention d'une numération de globules blancs (WBC) pour l'échantillon.

13. Procédé selon l'une quelconque des revendications 1 à 12, comprenant en outre la lyse des érythrocytes dans l'échantillon.

14. Procédé selon l'une quelconque des revendications 1 à 13, comprenant en outre le dépôt de cellules nucléées provenant de l'échantillon sous la forme d'une monocouche sur une lame de verre ;
facultativement comprenant en outre le dépôt de 3 millions de cellules provenant de l'échantillon sur ladite lame de verre.

15. Procédé selon l'une quelconque des revendications 2 à 14, dans lequel ledit nombre prédéterminé de CMC est au moins de 0,5, 1,0, 1,5, 2,0, 2,5, 3,0, 4,0, 5,0, 10, 20, 50, 100, 200, 300, 400 ou 500 CMC par ml d'échantillon.
